# EUROPÄISCHE PATENTSCHRIFT

(11) **EP 2 729 476 B1**
(45) Veröffentlichungstag und Bekanntmachung des Hinweises auf die Patenterteilung: **23.08.2017**
(21) Anmeldenummer: 12735116.1
(22) Anmeldetag: 05.07.2012
(51) Int. Cl.: A61K 31/519

(54) **HETEROARYL-SUBSTITUIERTE PYRAZOLOPYRIDINE UND IHRE VERWENDUNG ALS STIMULATOREN DER LÖSLICHEN GUANYLATCYCLASE**
HETEROARYL SUBSTITUTED PYRAZOLOPYRIDINES AND THEIR USE AS STIMULATORS OF SOLUBLE GUANYLATE CYCLASE
PYRAZOLOPYRIDINES HÉTÉROARYL SUBSTITUÉES ET LEUR UTILISATION EN TANT QUE STIMULATEURS DE LA GUANYLATE CYCLASE SOLUBLE

(30) Priorität: 06.07.2011 DE 102011078715; 11.01.2012 DE 102012200354
(43) Veröffentlichungstag der Anmeldung: 14.05.2014
(73) Patentinhaber: Bayer Intellectual Property GmbH, 40789 Monheim (DE)
(72) Erfinder: FOLLMANN, Markus, 50859 Wuppertal (DE); STASCH, Johannes-Peter, 42651 Solingen (DE); REDLICH, Gorden, 44799 Bochum (DE); GRIEBENOW, Nils, 41541 Dormagen (DE); LANG, Dieter, 42553 Velbert (DE); WUNDER, Frank, 42117 Wuppertal (DE)
(74) Vertreter: BIP Patents
(86) Internationale Anmeldenummer: PCT/EP2012/063155
(87) Internationale Veröffentlichungsnummer: WO 2013/004785

(56) Entgegenhaltungen:
- WO-A1-03/095451
- WO-A1-2010/065275
- WO-A1-2011/149921
- WO-A2-98/16507

## Beschreibung

Die vorliegende Anmeldung betrifft neue Heteroaryl-substituierte Pyrazolopyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart. WO 2010/065275 und WO 2011/149921 offenbaren substituierte Pyrrolo- und Dihydropyridopyrimidine als sGC Aktivatoren. 3-Furylindazole mit Heteroaryl-Substituenten in 1-Position als sGC Stimulatoren werden in Straub A. et al., Bioorg. Med. Chem. Lett. 11 (2001), 781-784 und WO 98/16507 beschrieben.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Offenbarungsgegenstand sind Verbindungen der allgemeinen Formel (I) 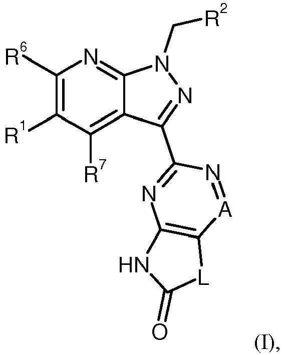 in welcher
- A: für Stickstoff oder CR³ steht,
wobei
R³ für Wasserstoff, Deuterium, Halogen, Difluormethyl, Trifluormethyl, (C₁-C₄)-Alkyl, (C₂-C₄)-Alkenyl, (C₂-C₄)-Alkinyl, Cyclopropyl, Cyclobutyl, Hydroxy, Amino, Phenyl oder 5- oder 6-gliedriges Heteroaryl steht,
worin (C₁-C₄)-Alkyl, (C₂-C₄)-Alkenyl, (C₂-C₄)-Alkinyl, Phenyl und 5- oder 6-gliedriges Heteroaryl mit 1 bis 3 Substituenten unabhängig voneinander ausgewählt aus der Gruppe mit Fluor, Difluormethyl, Trifluormethyl, (C₁-C₄)-Alkyl, Difluormethoxy, Trifluormethoxy, (C₁-C₄)-Alkoxy, (C₁-C₄)-Alkoxycarbonyl, Cyclopropyl und Cyclobutyl substituiert sein kann,
- L: für eine Gruppe *-CR^{4A}R^{4B}-(CR^{5A}R^{5B})ₚ₋# steht,
wobei
* für die Anknüpfstelle an die Carbonylgruppe steht,
# für die Anknüpfstelle an den Pyrimidinring bzw Triazinring steht,
p für eine Zahl 0, 1 oder 2 steht,
R^{4A} für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
R^{4B} für Wasserstoff, Fluor, Difluormethyl, Trifluormethyl, (C₁-C₆)-Alkyl, (C₁-C₄)-Alkoxycarbonylamino, Cyano, (C₃-C₇)-Cycloalkyl, Difluormethoxy, Trifluormethoxy, Phenyl oder eine Gruppe der Formel -M-R⁸ steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl und Amino substituiert sein kann,
und worin
M für eine Bindung oder (C₁-C₄)-Alkandiyl steht,
- R⁸: für -(C=O)ᵣ-OR⁹, -(C=O),-NR⁹R¹⁰, -C(=S)-NR⁹R¹⁰, -NR⁹-(C=O)-R¹², -NR⁹-(C=O)-NR¹⁰R¹¹, -NR⁹-SO₂-NR¹⁰R¹¹, -NR⁹-SO₂-R¹², -S(O)ₛ-R¹², -SO₂-NR⁹R¹⁰, 4- bis 7-gliedriges Heterocyclyl, Phenyl oder 5- oder 6-gliedriges Heteroaryl steht,
worin
r die Zahl 0 oder 1 bedeutet,
s die Zahl 0, 1 oder 2 bedeutet,
R⁹, R¹⁰ und R¹¹ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen, oder
R⁹ und R¹⁰ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
R¹⁰ und R¹¹ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹² für (C₁-C₆)-Alkyl oder (C₃-C₇)-Cycloalkyl steht,
oder
R⁹ und R¹² bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, und
worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen, sofern nicht anders angegeben, jeweils unabhängig voneinander weiterhin mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder
- R^{4A} und R^{4B}: zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
R^{5A} für Wasserstoff, Fluor, (C₁-C₄)-Alkyl, (C₁-C₄)-Alkoxycarbonyl oder Hydroxy steht,
R^{5B} für Wasserstoff, Fluor, (C₁-C₄)-Alkyl oder Trifluormethyl steht,
- R¹: für Wasserstoff, Halogen, Cyano, Difluormethyl, Trifluormethyl, (C₁-C₄)-Alkyl oder (C₃-C₇)-Cycloalkyl steht,
- R²: für 5- oder 6-gliedriges Heteroaryl steht,
wobei 5- und 6-gliedriges Heteroaryl mit 1 oder 2 Substituenten Fluor substituiert sein können,
- R⁶: für Wasserstoff, Cyano, Difluormethyl, Trifluormethyl, (C₁-C₄)-Alkyl oder (C₃-C₇)-Cycloalkyl steht,
- R⁷: für Wasserstoff, Cyano, Difluormethyl, Trifluormethyl, (C₁-C₄)-Alkyl oder (C₃-C₇)-Cycloalkyl steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren *N*-Oxide, Salze, Solvate und Solvate der *N*-Oxide und Salze, die von Formel (I) umfassten Verbindungen der nachfolgend genannten Formeln und deren *N*-Oxide, Salze, Solvate und Solvate der *N*-Oxide und Salze sowie die von Formel (I) umfassten, nachfolgend als Ausführungsbeispiele genannten Verbindungen und deren *N*-Oxide, Salze, Solvate und Solvate der *N*-Oxide und Salze, soweit es sich bei den von Formel (I) umfassten, nachfolgend genannten Verbindungen nicht bereits um N-Oxide, Salze, Solvate und Solvate der *N*-Oxide und Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, lag, ¹⁵ N, ¹⁷O, ¹⁸O, ³²P, ³³P, ³³S, ³⁴S, ³⁵S, ³⁶S, ¹⁸F, ³⁶Cl, ⁸² Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.

Alkandi^{y}l steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-diyl, Propan-1,2-diyl, Propan-2,2-diyl, Butan-1,4-diyl, Butan-1,2-diyl, Butan-1,3-diyl und Butan-2,3-diyl.

Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy,

Alkoxycarbonyl steht im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.

Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.

Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N-*Dimethylamino, *N*,*N*-Diethylamino, *N*-Ethyl-*N*-methylamino, *N*-Methyl-*N*-n-propylamino, *N*-Isopropyl-*N*-n-propylamino, *N*-tert.-Butyl-N-methylamino, *N*-Ethyl-*N*-n-pentylamino und *N*-n-Hexyl-*N*-methylamino.

Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.

5- oder 6- gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.

8- oder 9-gliedriges Heteroaryl steht im Rahmen der Erfindung für einen bicyclischen aromatischen oder teilweise ungesättigten Heterocyclus mit insgesamt 8 oder 9 Ringatomen, der mindestens zwei Stickstoffatome und bis zu zwei weitere, gleiche oder verschiedene Ring-Heteroatome aus der Reihe N, O und/oder S enthält. Beispielhaft seien genannt: Dihydrothienopyrazolyl, Thienopyrazolyl, Pyrazolopyrazolyl, Imidazothiazolyl, Tetrahydrocyclopentapyrazolyl, Dihydrocyclopentapyrazolyl, Tetrahydroindazolyl, Dihydroindazolyl, Indazolyl, Pyrazolopyridinyl, Tetrahydropyrazolopyridinyl, Pyrazolopyrimidinyl, Imidazopyridinyl und Imidazopyridazinyl.

Halogen steht im Rahmen der Erfindung für Fluor, Chlor, Brom und Iod. Bevorzugt sind Brom und Iod.

Eine Oxo-Gruppe steht im Rahmen der Erfindung für ein Sauerstoffatom, das über eine Doppelbindung an ein Kohlenstoffatom gebunden ist.

Eine Thiooxo-Gruppe steht im Rahmen der Erfindung für ein Schwefelatom, das über eine Doppelbindung an ein Kohlenstoffatom gebunden ist.

In der Formel der Gruppe, für die L stehen kann, steht der Endpunkt der Linie, an dem das Zeichen * und # steht, nicht für ein Kohlenstoffatom beziehungsweise eine CH₂-Gruppe, sondern ist Bestandteil der Bindung zu dem jeweils bezeichneten Atom, an das L gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die Verbindungen der Formel (I-1) bilden eine Untergruppe der erfindungsgemäßen Verbindungen der Formel (I), in welcher R⁶ und R⁷ für Wasserstoff stehen.

Ebenfalls offenbart sind Verbindungen der Formel (I), in welcher
- A: für Stickstoff oder CR³ steht,
wobei
R³ für Wasserstoff, Deuterium, Fluor, Iod, Difluormethyl, Trifluormethyl, (C₁-C₄)-Alkyl, Vinyl, Allyl, Ethinyl, Cyclopropyl, Cyclobutyl, Hydroxy, Pyrazolyl oder Pyridyl steht,
worin (C₁-C₄)-Alkyl, Vinyl, Allyl, Ethinyl und Pyridyl mit 1 oder 2 Substituenten unabhängig voneinander ausgewählt aus der Gruppe mit Methyl, Cyclopropyl und Cyclobutyl substituiert sein können,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring bzw Triazinring steht,
p für eine Zahl 0, 1 oder 2 steht,
R^{4A} für Wasserstoff, Fluor, Methyl, Ethyl, Hydroxy oder Amino steht,
R^{4B} für Wasserstoff, Fluor, Difluormethyl, Trifluormethyl, (C₁-C₄)-Alkyl, Methoxy-carbonylamino, Cyano, Cyclopropyl, Cyclobutyl, Cyclopentyl, Phenyl oder eine Gruppe der Formel -M-R⁸ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung oder Methylen steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander
ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Thienyl, Pyridyl, Pyrimidinyl, Pyrazinyl oder Pyridazinyl steht,
wobei Thienyl, Pyridyl, Pyrimidinyl, Pyrazinyl und Pyridazinyl mit 1 oder 2 Substituenten Fluor substituiert sein können,
- R⁶: für Wasserstoff oder Methyl steht,
- R⁷: für Wasserstoff steht,
sowie ihre Salze, Solvate und Solvate der Salze.

Ebenfalls offenbart sind auch Verbindungen der Formel (I), in welcher
- A: für Stickstoff oder CR³ steht,
wobei
R³ für Wasserstoff, Fluor, Difluormethyl, Trifluormethyl, Methyl, Ethyl, Cyclopropyl, oder Cyclobutyl steht,
- L: für eine Gruppe *-CR^{4A}R^{4B}-(CR^{5A}R^{5B})ₚ-# steht, wobei
- *: für die Anknüpfstelle an die Carbonylgruppe steht,
- #: für die Anknüpfstelle an den Pyrimidinring bzw Triazinring steht,
- p: für eine Zahl 0 steht,
- R^{4A}: für Wasserstoff, Fluor, Methyl, Ethyl, Hydroxy oder Amino steht,
- R^{4B}: für Wasserstoff, Fluor, Difluormethyl, Trifluormethyl, Methyl, Ethyl, Methoxy-carbonylamino, Cyclopropyl, Cyclobutyl, Cyclopentyl oder eine Gruppe der Formel -M-R⁸ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung steht,
R⁸ für -(C=O)ᵣ-NR⁹R¹⁰, Phenyl, Thiazolyl, Triazolyl, Oxadiazolyl, Thiadiazolyl oder Pyrimidinyl steht,
worin
r die Zahl 1 bedeutet,
R⁹ und R¹⁰ unabhängig voneinander jeweils für Wasserstoff, oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für Thienyl, Pyridyl oder Pyrimidinyl steht,
wobei Thienyl, Pyridyl und Pyrimidinyl mit 1 oder 2 Substituenten Fluor substituiert sein können,
- R⁶: für Wasserstoff oder Methyl steht,
- R⁷: für Wasserstoff steht,
sowie ihre Salze, Solvate und Solvate der Salze.

Gegenstand der vorliegenden Erfindung sind Verbindungen der Formel (I), in welcher
- A: für Stickstoff oder CR³ steht,
wobei
R³ für Wasserstoff oder Amino steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring bzw Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
- R¹: für Wasserstoff oder Fluor steht,
- R²: für Thienyl, Pyridyl oder Pyrimidinyl steht,
wobei Thienyl, Pyridyl und Pyrimidinyl mit 1 oder 2 Substituenten Fluor substituiert sein können,
- R⁶: für Wasserstoff steht,
- R⁷: für Wasserstoff steht,
sowie ihre Salze, Solvate und Solvate der Salze.

Ebenfalls offenbart sind auch Verbindungen der Formel (I-1), in welcher 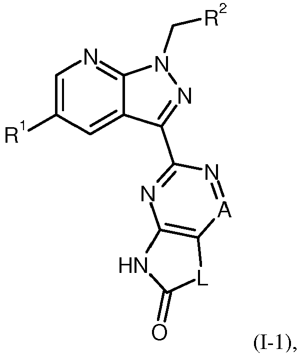 in welcher
- A: für Stickstoff oder CR³ steht,
wobei
R³ für Wasserstoff, Deuterium, Halogen, Difluormethyl, Trifluormethyl, (C₁-C₄)-Alkyl, Cyclopropyl, Cyclobutyl, Hydroxy oder Amino steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
R^{4B} für Wasserstoff, Fluor, (C₁-C₄)-Alkyl, Trifluormethyl, (C₁-C₄)-Alkoxycarbonyl-amino oder Phenyl steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann, oder
R^{4A} und R^{4B} zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können, oder
R^{4A} und R^{4B} zusammen mit dem Kohlenstoffatom, an das sie gebunden sind, eine (C₂-C₄)-Alkenyl-Gruppe bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
- R¹: für Wasserstoff oder Fluor steht,
- R²: für 5- oder 6-gliedriges Heteroaryl steht, wobei 5- und 6-gliedriges Heteroaryl mit 1 oder 2 Substituenten Fluor substituiert sein können,
sowie ihre *N*-Oxide, Salze, Solvate, Salze der *N*-Oxide und Solvate der *N*-Oxide und Salze.

Ebenfalls offenbart sind Verbindungen der Formel (I-1), in welcher
- A: für Stickstoff oder CR³ steht, wobei
R³ für Wasserstoff, Deuterium, Fluor, Iod, Difluormethyl, Trifluormethyl, (C₁-C₄)-Alkyl, Cyclopropyl, Cyclobutyl oder Hydroxy steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0, 1 oder 2 steht,
R^{4A} für Wasserstoff, Fluor, Methyl, Ethyl oder Hydroxy steht,
R^{4B} für Wasserstoff, Fluor, Methyl, Ethyl oder Trifluormethyl steht, oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Pyridyl, Pyrimidinyl, Pyrazinyl oder Pyridazinyl steht,
wobei Pyridyl, Pyrimidinyl, Pyrazinyl oder Pyridazinyl mit 1 oder 2 Substituenten Fluor substituiert sein können,
sowie ihre Salze, Solvate und Solvate der Salze.

Ebenfalls offenbart sind Verbindungen der Formel (I-1), in welcher
- A: für CR³ steht,
wobei
R³ für Amino steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring bzw Triazinringsteht,
p für eine Zahl 0, 1 oder 2 steht,
R^{4A} für Wasserstoff, Fluor, Methyl, Ethyl oder Hydroxy steht,
R^{4B} für Wasserstoff, Fluor, Methyl, Ethyl oder Trifluormethyl steht, oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetra-hydrofuranyl-, Piperidinyl- oder Tetrahydropyranyl-Ring bilden,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für Pyridyl, Pyrimidinyl, Pyrazinyl oder Pyridazinyl steht,
wobei Pyridyl, Pyrimidinyl, Pyrazinyl oder Pyridazinyl mit 1 oder 2 Substituenten Fluor substituiert sein können,
sowie ihre Salze, Solvate und Solvate der Salze.

Ebenfalls offenbart sind Verbindungen der Formel (I-1), in welcher
- A: für CR³ steht,
wobei
R³ für Wasserstoff steht,
- L: für eine Gruppe *-CR^{4A}R^{4B}-(CR^{5A}R^{5B})ₚ-# steht, wobei
* für die Anknüpfstelle an die Carbonylgruppe steht,
# für die Anknüpfstelle an den Pyrimidinring steht,
p für eine Zahl 0 oder 1 steht,
R^{4A} für Wasserstoff, Methyl oder Hydroxy steht,
R^{4B} für Wasserstoff, Fluor, Methyl oder Trifluormethyl steht, oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für 3-Fluorpyrid-2-yl oder Pyrimidin-2-yl steht,
sowie ihre Salze, Solvate und Solvate der Salze.

Ebenfalls offenbart sind auch Verbindungen der Formel (I-1), in welcher
- A: für Stickstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 oder 1 steht,
R^{4A} für Wasserstoff, Methyl oder Hydroxy steht,
R^{4B} für Wasserstoff, Fluor, Methyl oder Trifluormethyl steht, oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
R¹ für Wasserstoff oder Fluor steht,
R² für 3-Fluorpyrid-2-yl oder Pyrimidin-2-yl steht,
sowie ihre Salze, Solvate und Solvate der Salze.

Bevorzugt sind im Rahmen der vorliegenden Erfindung auch Verbindungen der Formel (I) und (I-1), in welcher R¹ für H steht, sowie ihre Salze, Solvate und Solvate der Salze.

Bevorzugt sind im Rahmen der vorliegenden Erfindung auch Verbindungen der Formel (I) und (I-1), in welcher R¹ für Fluor steht, sowie ihre Salze, Solvate und Solvate der Salze.

Bevorzugt sind im Rahmen der vorliegenden Erfindung auch Verbindungen der Formel (I) und (I-1), in welcher A für N oder CH steht, sowie ihre Salze, Solvate und Solvate der Salze.

Bevorzugt sind im Rahmen der vorliegenden Erfindung auch Verbindungen der Formel (I) und (I-1), in welcher A für N steht, sowie ihre Salze, Solvate und Solvate der Salze.

Bevorzugt sind im Rahmen der vorliegenden Erfindung auch Verbindungen der Formel (I) und (I-1), in welcher A für CH steht, sowie ihre Salze, Solvate und Solvate der Salze.

Ebenfalls offenbart sind auch Verbindungen der Formel (I) und (I-1), in welcher
- A: für Stickstoff oder CR³ steht,
wobei
R³ für Wasserstoff, Deuterium, Fluor, Iod, Difluormethyl, Trifluormethyl, (C₁-C₄)-Alkyl, Cyclopropyl, Cyclobutyl oder Hydroxy steht,
sowie ihre Salze, Solvate und Solvate der Salze.

Ebenfalls offenbart sind Verbindungen der Formel (I) und (I-1), in welcher
- A: für CR³ steht,
wobei
- R³: für Wasserstoff, Deuterium, Fluor, Iod, Difluormethyl, Trifluormethyl, (C₁-C₄)-Alkyl, Cyclopropyl, Cyclobutyl oder Hydroxy steht,
sowie ihre Salze, Solvate und Solvate der Salze.

Bevorzugt sind im Rahmen der vorliegenden Erfindung auch Verbindungen der Formel (I) und (I-1), in welcher
- A: für CR³ steht,
wobei
R³ für Amino steht,
sowie ihre Salze, Solvate und Solvate der Salze.

Bevorzugt sind im Rahmen der vorliegenden Erfindung auch Verbindungen der Formel (I) und (I-1), in welcher
- A: für CR³ steht,
wobei
R³ für Wasserstoff steht,
- L: für eine Gruppe *-CR^{4A}R^{4B}-(CR^{5A}R^{5B})ₚ-# steht
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
sowie ihre Salze, Solvate und Solvate der Salze.

Bevorzugt sind im Rahmen der vorliegenden Erfindung auch Verbindungen der Formel (I) und (I-1), in welcher
- A: für CR³ steht,
wobei
R³ für Wasserstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Methyl steht,
R^{4B} für Methyl steht,
sowie ihre Salze, Solvate und Solvate der Salze.

Bevorzugt sind im Rahmen der vorliegenden Erfindung auch Verbindungen der Formel (I) und (I-1), in welcher
- A: für Stickstoff steht,
- L: für eine Gruppe *-CR^{4A}R⁴B-(CR^{5A}R^{5B})ₚ-# steht
wobei
* für die Anknüpfstelle an die Carbonylgruppe steht,
# für die Anknüpfstelle an den Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
sowie ihre Salze, Solvate und Solvate der Salze.

Bevorzugt sind im Rahmen der vorliegenden Erfindung auch Verbindungen der Formel (I) und (I-1), in welcher
- A: für Stickstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 steht,
R^{4A} für Methyl steht,
R^{4B} für Methyl steht,
sowie ihre Salze, Solvate und Solvate der Salze.

Ebenfalls offenbart sind Verbindungen der Formel (I) und (I-1), in welcher
- L: für eine Gruppe *-CR^{4A}R^{4B}-(CR^{5A}R^{5B})ₚ-# steht
wobei
* für die Anknüpfstelle an die Carbonylgruppe steht,
# für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetra-hydrofuranyl-, Piperidinyl- oder Tetrahydropyranyl-Ring bilden,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe *-CR^{4A}R^{4B}-(CR^{5A}R^{5B})ₚ-# steht
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
sowie ihre Salze, Solvate und Solvate der Salze.

Bevorzugt sind im Rahmen der vorliegenden Erfindung auch Verbindungen der Formel (I) und (I-1), in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} für Methyl steht,
R^{4B} für Methyl steht,
sowie ihre Salze, Solvate und Solvate der Salze.

Ebenfalls offenbart sind auch Verbindungen der Formel (I) und (I-1), in welcher
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
sowie ihre Salze, Solvate und Solvate der Salze.

Ebenfalls offenbart sind Verbindungen der Formel (I) und (I-1), in welcher
- R²: für Furyl, Pyrrolyl, Thienyl, Pyrazolyl, Imidazolyl, Thiazolyl, Oxazolyl, Isoxazolyl, Iso-thiazolyl, Triazolyl, Oxadiazolyl, Thiadiazolyl, Pyridyl, Pyrimidinyl, Pyridazinyl, Pyrazinyl oder Triazinyl steht,
wobei Furyl, Pyrrolyl, Thienyl, Pyrazolyl, Imidazolyl, Thiazolyl, Oxazolyl, Isoxazolyl, Iso-thiazolyl, Triazolyl, Oxadiazolyl, Thiadiazolyl, Pyridyl, Pyrimidinyl, Pyridazinyl, Pyrazinyl und Triazinyl mit 1 oder 2 Substituenten Fluor substituiert sein können,
sowie ihre Salze, Solvate und Solvate der Salze.

Ebenfalls offenbart sind auch Verbindungen der Formel (I) und (I-1), in welcher
- R²: für Pyridyl, Pyrimidinyl, Pyridazinyl, Pyrazinyl oder Triazinyl steht,
wobei Pyridyl, Pyrimidinyl, Pyridazinyl und Pyrazinyl mit 1 oder 2 Substituenten Fluor substituiert sein können,
sowie ihre Salze, Solvate und Solvate der Salze.

Ebenfalls offenbart sind auch Verbindungen der Formel (I) und (I-1), in welcher
- R²: für Pyrid-2-yl, Pyrid-4-yl, Pyrimidin-2-yl, Pyrimidin-5-yl oder Pyrazin-2-yl steht,
wobei Pyrid-2-yl und Pyrid-4-yl mit 1 oder 2 Substituenten Fluor substituiert sein können,
sowie ihre Salze, Solvate und Solvate der Salze.

Bevorzugt sind im Rahmen der vorliegenden Erfindung auch Verbindungen der Formel (I) und (I-1), in welcher
- R²: für 3-Fluorpyrid-2-yl oder Pyrimidin-2-yl steht,
sowie ihre Salze, Solvate und Solvate der Salze.

Bevorzugt sind im Rahmen der vorliegenden Erfindung auch Verbindungen der Formel (I) und (I-1), in welcher
- R²: für 3-Fluorpyrid-2-yl steht,
sowie ihre Salze, Solvate und Solvate der Salze.

Ebenfalls offenbart sind auch Verbindungen der Formel (I), in welcher
- A: für CR³ steht,
wobei
R³ für Wasserstoff steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring steht,
p für eine Zahl 0 steht,
R^{4A} für Wasserstoff, Fluor, Methyl, Ethyl, Hydroxy oder Amino steht,
R^{4B} für eine Gruppe der Formel -M-R⁸ steht,
worin
M für eine Bindung steht,
R⁸ für -(C=O),-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Ebenfalls offenbart sind auch Verbindungen der Formel (I), in welcher
- A: für N steht,
- L: für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Triazinring steht,
p für eine Zahl 0 steht,
R^{4A} für Wasserstoff, Fluor, Methyl, Ethyl, Hydroxy oder Amino steht,
R^{4B} für eine Gruppe der Formel -M-R⁸ steht,
worin
M für eine Bindung steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 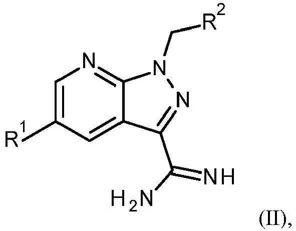 in welcher R¹ und R² jeweils die oben genannten Bedeutungen haben,
[A] in einem inerten Lösungsmittel in Gegenwart einer geeigneten Base mit einer Verbindung der Formel (III) 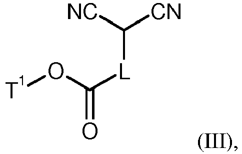
   in welcher L die oben genannte Bedeutung hat und
      - T¹: für (C₁-C₄)-Alkyl steht,
   zu einer Verbindung der Formel (IV) 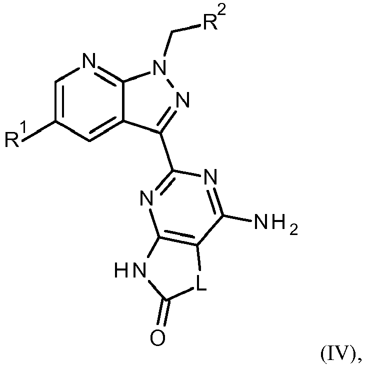
   in welcher L, R¹ und R² jeweils die oben angegebenen Bedeutungen haben,
   umsetzt, diese dann mit iso-Pentylnitrit und einem Halogen-Äquivalent in eine Verbindung der Formel (V) 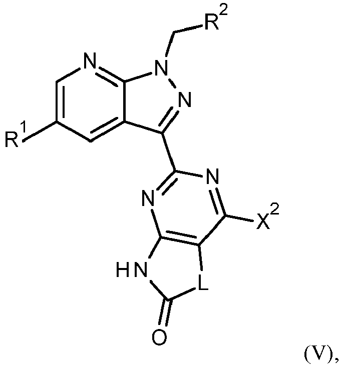
   in welcher L, R¹ und R² jeweils die oben angegebenen Bedeutungen haben und
      - X²: für Brom oder Iod steht,
   überführt, und diese im Anschluss in einen inerten Lösungsmittel in Gegenwart eines geeigneten Übergangsmetallkatalysators zu einer Verbindung der Formel (I-A) 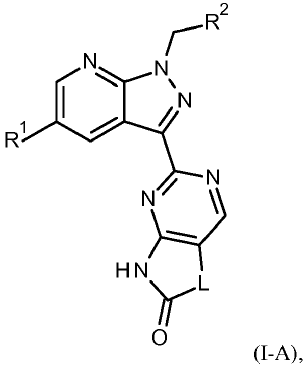
   in welcher L, R¹ und R² jeweils die oben angegebenen Bedeutungen haben,
   umsetzt,
   oder
[B] in einem inerten Lösungsmittel in Gegenwart einer geeigneten Base mit Hydrazinhydrat zu einer Verbindung der Formel (VI) 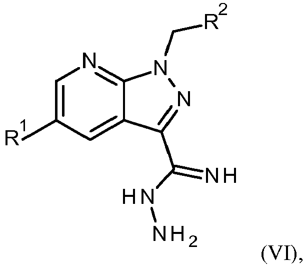
   in welcher R¹ und R² jeweils die oben genannten Bedeutungen haben,
   umsetzt, diese dann in einem inerten Lösungsmittel mit einer Verbindung der Formel (VII) 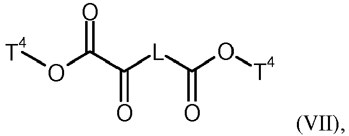
   in welcher L die oben angegebene Bedeutung hat und
      - T⁴: für (C₁-C₄)-Alkyl steht,
   zu einer Verbindung der Formel (VIII) 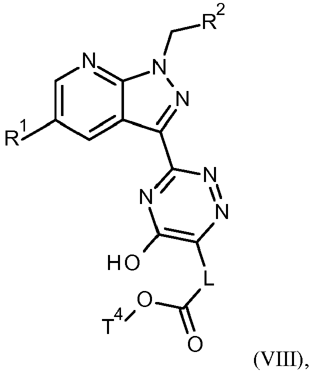
   in welcher L, R¹ und R² und T⁴ jeweils die oben angegebenen Bedeutungen haben,
   reagiert, diese anschliessend mit Phosphorylchlorid in eine Verbindung der Formel (IX) 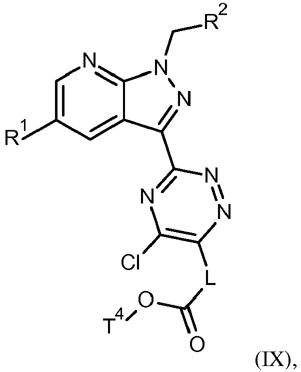
   in welcher L, R¹ und R² und T⁴ jeweils die oben angegebenen Bedeutungen haben,
   überführt, und diese direkt mit Ammoniak zu einer Verbindung der Formel (X) 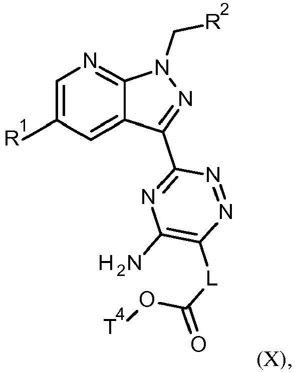
   in welcher L, R¹ und R² und T⁴ jeweils die oben angegebenen Bedeutungen haben,
   umsetzt, und schliesslich in einem inerten Lösungsmittel gegebenenfalls in Gegenwart einer geeigneten Base zu einer Verbindung der Formel (I-B) 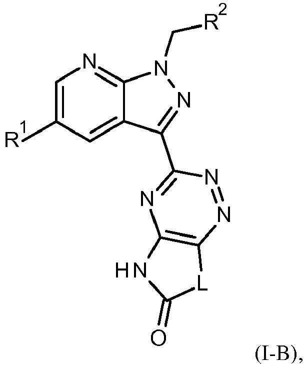
   in welcher L, R¹ und R² jeweils die oben angegebenen Bedeutungen haben,
   cyclisiert,
und gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A) und (I-B) bilden zusammen die Gruppe der erfindungsgemäßen Verbindungen der Formel (I).

Inerte Lösungsmittel für den Verfahrensschritt (II) + (III) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol oder Methanol.

Geeignete Basen für den Verfahrensschritt (II) + (III) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat oder Natriummethanolat.

Die Reaktion (II) + (III) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (IV) → (V) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (IV) → (V) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (IV) → (V) eignen sich beispielsweise Diiodmethan, eine Mischung aus Cäsiumiodid, Iod und Kupfer-(I)-iodid oder Kupfer -(II)-bromid.

Der Verfahrensschritt (IV) → (V) erfolgt im Fall von Diiodmethan als Halogenquelle mit einem Molverhältnis von 10 bis 30 Mol Isopentylnitrit und 10 bis 30 Mol des Iod-Äquivalents bezogen auf 1 Mol der Verbindung der Formel (IV).

Inerte Lösungsmittel für den Verfahrensschritt (V) → (I-A) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), *N*,*N*'-Dimethylpropylenharnstoff (DMPU), *N-*Methylpyrrolidon (NMP), Pyridin oder auch Wasser. Ebenso ist es möglich, Gemische der genannten Lösungsmittel einzusetzen. Bevorzugt ist DMF.

Die Reduktion (V) → (I-A) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (V) → (I-A) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzung (VIII) → (IX) kann in einem unter den Reaktionsbedingungen inerten Lösungsmittel oder ohne Lösungsmittel erfolgen. Bevorzugtes Lösungsmittel ist Sulfolan.

Die Reaktion (VIII) → (IX) erfolgt im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (VIII) → (IX) ohne Lösungsmittel in einem Temperaturbereich von 0°C bis +50°C bei Normaldruck.

Der Verfahrensschritt (IX) → (X) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (IX) → (X) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Cyclisierung (X) → (I-B) erfolgt in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N-*Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für den Verfahrensschritt (X) → (I-B) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (X) → (I-B) erfolgt im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgt die Cyclisierung zu (I-B) direkt bei der Umsetzung (IX) → (X) ohne Zugabe weiterer Reagenzien.

In einer alternativen Durchführung des Verfahrens [B] wird die Umwandlung (VI) + (VII) → (VIII) → (IX) → (X) → (I-B) ohne Isolierung der Zwischenstufen durchgeführt.

Bevorzugt erfolgen die Umsetzungen (VIII) → (IX) → (X) → (I-B) ohne Isolierung der Zwischenstufen.

Inerte Lösungsmittel für den Verfahrensschritt (VI) + (VII) → (VIII) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (VI) + (VII) → (VIII) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (II) → (VI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Ethanol.

Geeignete Basen für den Verfahrensschritt (II) → (VI) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt Triethylamin.

Die Reaktion (II) → (VI) erfolgt im Allgemeinen in einem Temperaturbereich von 0°C bis +60°C, bevorzugt von +10°C bis +30°C. Die Umsetzung kann bei normalem oder erhöhtem Druck durchgeführt werden (z.B. im Bereich von 0.5 bis 5 bar). Im Allgemeinen arbeitet man bei Normaldruck.

Die beschriebenen Herstellverfahren können durch die folgenden Syntheseschemata (Schema 1 und 2) beispielhaft verdeutlicht werden: 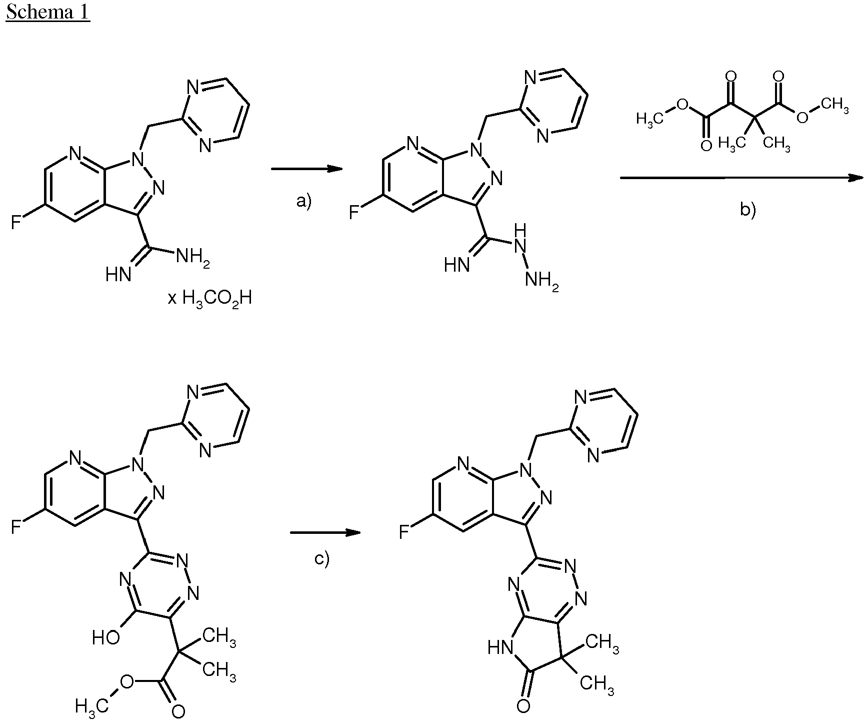 [a): Hydrazinhydrat, NEt₃, EtOH b): EtOH c): 1. POCl₃; 2. konz. NH₃, Acetonitril]. 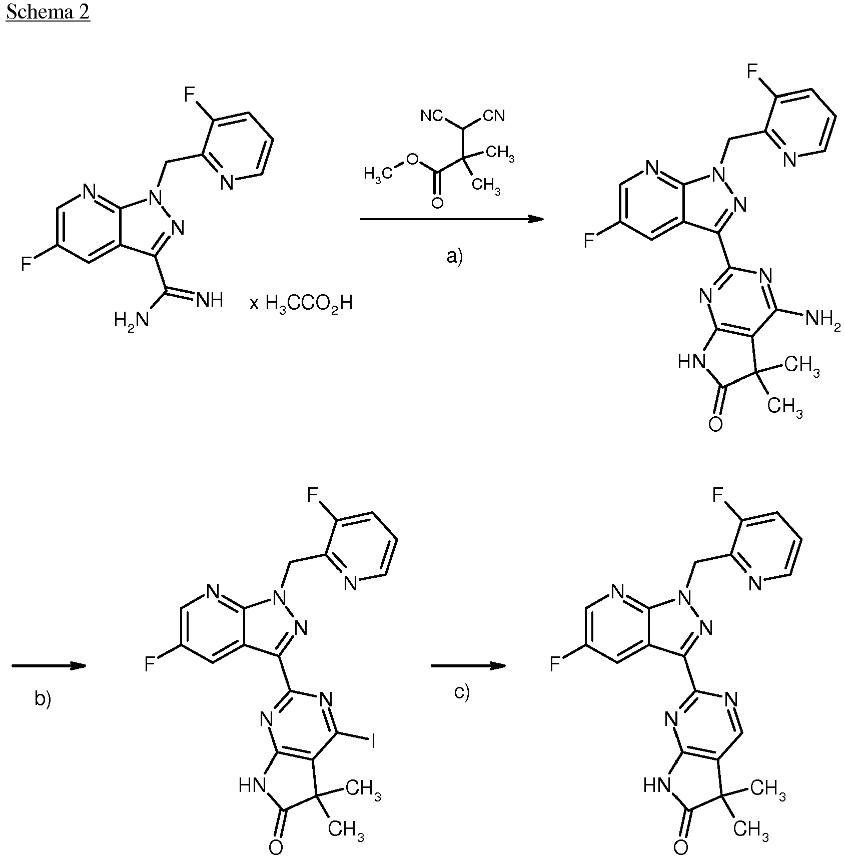 [a): KOt-Bu, tert.-Butanol; b): Diiodmethan, iso-Pentylnitrit; c): Pd/C, Wasserstoff, DMF].

Weitere erfindungsgemäße Verbindungen können gegebenenfalls auch hergestellt werden durch Umwandlungen von funktionellen Gruppen einzelner Substituenten, insbesondere den unter L und R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

In einem alternativen Verfahren kann die Herstellung der erfindungsgemäßen Verbindungen der Formel (I) durch Umkehrung der Reaktitionsschritte unter Verwendung von Schutzgruppenchemie erfolgen, wie im nachfolgenden Syntheseschema (Schema 6) beispielhaft gezeigt: 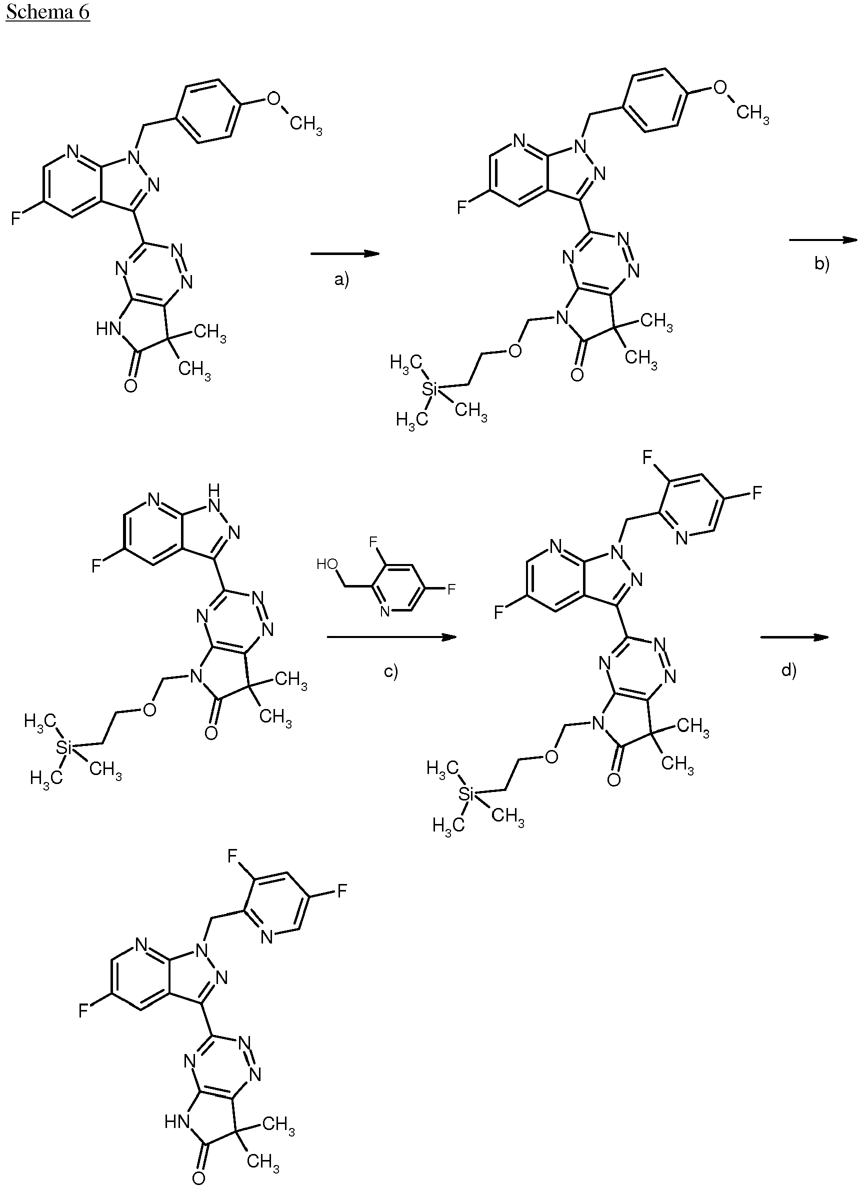 [a): 2-(Trimethylsilyl)ethoxymethylchlorid, Cs₂CO₃, DMF; b): Ammoniumcer(IV)-nitrat, Acetonitril, Wasser; c): Diisopropylazodicarboxylat, Triphenylphosphin, THF, Dichlormethan; d): 1) TFA, Dichlormethan, 2) HCl, Ethanol].

Die Verbindungen der Formel (II) können hergestellt werden, indem man eine Verbindung der Formel (XI) 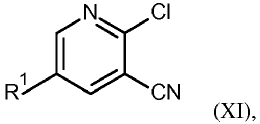 in welcher R¹ die oben angegebene Bedeutung hat,
in einem inerten Lösungsmittel mit Hydrazinhydrat zur Verbindung der Formel (XII) 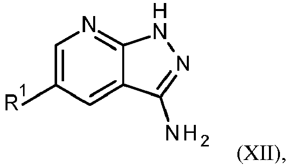 in welcher R¹ die oben angegebene Bedeutung hat,
zyklisiert, diese anschliessend in einem inerten Lösungsmittel in Gegenwart einer geeigneten Lewis-Säure zunächst mit Isopentylnitrit zum entsprechenden Diazoniumsalz umsetzt, und dieses dann direkt mit Natriumiodid in die Verbindung der Formel (XIII) 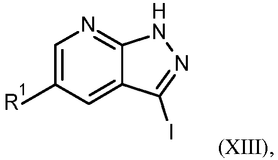 in welcher R¹ die oben angegebene Bedeutung hat,
überführt, diese im Folgenden in einem inerten Lösungsmittel in Gegenwart einer geeigneten Base mit der Verbindung der Formel (XIV) 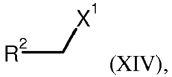 in welcher R² die oben angegebene Bedeutung hat und
- X¹: für eine geeignete Abgangsgruppe, wie beispielsweise Halogen, Tosylat oder Mesylat, steht, oder für Hydroxy steht,
in eine Verbindung der Formel (XV) 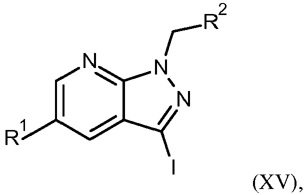 in welcher R¹ und R² jeweils die oben angegebenen Bedeutungen haben,
überführt, diese anschließend in einem inerten Lösungsmittel mit Kupfercyanid zu einer Verbindung der Formel (XVI) 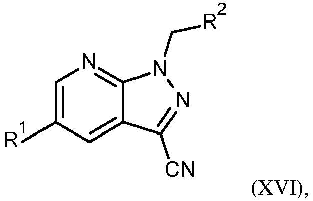 in welcher R¹ und R² jeweils die oben angegebenen Bedeutungen haben,
reagiert, und diese schließlich unter sauren Bedingungen mit einem Ammoniak-Äquivalent umsetzt.

Inerte Lösungsmittel für den Verfahrensschritt (XI) → (XII) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist 1,2-Ethandiol.

Die Reaktion (XI) → (XII) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XII) → (XIII) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Bevorzugt ist DMF.

Als Lewis-Säuren für den Verfahrensschritt (XII) → (XIII) eignen sich Bortrifluorid-Diethylether-Komplex, Cer(IV)ammoniumnitrat (CAN), Zinn(II)chlorid, Lithiumperchlorat, Zink(II)chlorid, Indium(III)chlorid oder Indium(III)bromid. Bevorzugt ist Bortrifluorid-Diethylether-Komplex.

Die Reaktion (XII) → (XIII) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XIII) + (XIV) → (XV) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril. Bevorzugt ist DMF.

Geeignete Basen für den Verfahrensschritt (XIII) + (XIV) → (XV) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Cäsiumcarbonat.

Die Reaktion (XIII) + (XIV) → (XV) wird im Allgemeinen in einem Temperaturbereich von 0°C bis +60°C, bevorzugt bei +10°C bis +25°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Wenn X² für Hydroxy steht, erfolgt die Umsetzung (XIII) + (XIV) → (XV) unter Mitsunobu-Bedingungen. Die Mitsunobu-Reaktion erfolgt unter Verwendung von Triphenylphosphin, oder Tri-n-butylphosphin, 1,2-Bis(diphenylphosphino)ethan (DPPE), Diphenyl(2-pyridyl)phosphin (Ph2P-Py), (p-Dimethylaminophenyl)diphenylphosphin (DAP-DP), tris(4-Dimethylaminophenyl)-phosphin (tris-DAP) und eines geeigneten Dialkylazodicarboxylats, wie beispielsweise Diethylazodicarboxylat (DEAD), Diisopropylazodicarboxylat (DIAD), Di-tert-butylazodicarboxylat, N,N,N'N'-Tetramethylazodicarboxamid (TMAD), 1,1'-(Azodicarbonyl)-dipiperidin (ADDP) oder 4,7-Dimethyl-3,5,7-hexahydro-1,2,4,7-tetrazocin-3,8-dion (DHTD). Bervorzugt werden Triphenylphosphin und Diisopropylazodicarboxylat (DIAD) verwendet, oder eines geeigneten Azodicarbonamides, wie beispielsweise N,N,N',N'-Tetramethyldiazen-1,2-dicarboxamid.

Inerte Lösungsmittel für die Mitsunobu-Reaktion (XIII) + (XIV) → (XV) sind beispielsweise Ether wie Tetrahydrofuran, Diethylether, Kohlenwasserstoffe wie Benzol, Toluol, Xylol, Halogenkohlenwasserstoffe wie Dichlormethan, Dichlorethan oder andere Lösungsmittel wie Acetonitril, DMF oder NMP. Ebenso ist es möglich, Gemische der genannten Lösungsmittel einzusetzen. Bevorzugt wird THF verwendet.

Die Mitsunobu-Reaktion (XIII) + (XIV) → (XV) erfolgt im Allgemeinen in einem Temperaturbereich von -78°C bis +180°C, bevorzugt bei 0°C bis +50°C, gegebenenfalls in einer Mikrowelle. Die Umsetzungen können bei normalem, erhöhtem oder bei erniedrigtem Druck durchgeführt werden (z.B. von 0.5 bis 5 bar).

Inerte Lösungsmittel für den Verfahrensschritt (XV) → (XVI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethyl-propylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (XV) → (XVI) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (XVI) → (II) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in einer geeigneten Säure unter Bildung des Amidins (III) bei +50 bis +150°C.

Geeignete Säure für die Bildung des Amidins (II) sind anorganische Säuren wie beispielsweise Chlorwasserstoff/ Salzsäure, Schwefelsäure, Polyphosphorsäure oder Phosphorsäure oder organische Säuren wie beispielsweise Essigsäure, Trifluoressigsäure oder Ameisensäure. Bervorzugt werden Salzsäure oder Essigsäure verwendet.

Das beschriebene Herstellverfahren kann durch das folgende Syntheseschema (Schema 3) beispielhaft verdeutlicht werden: 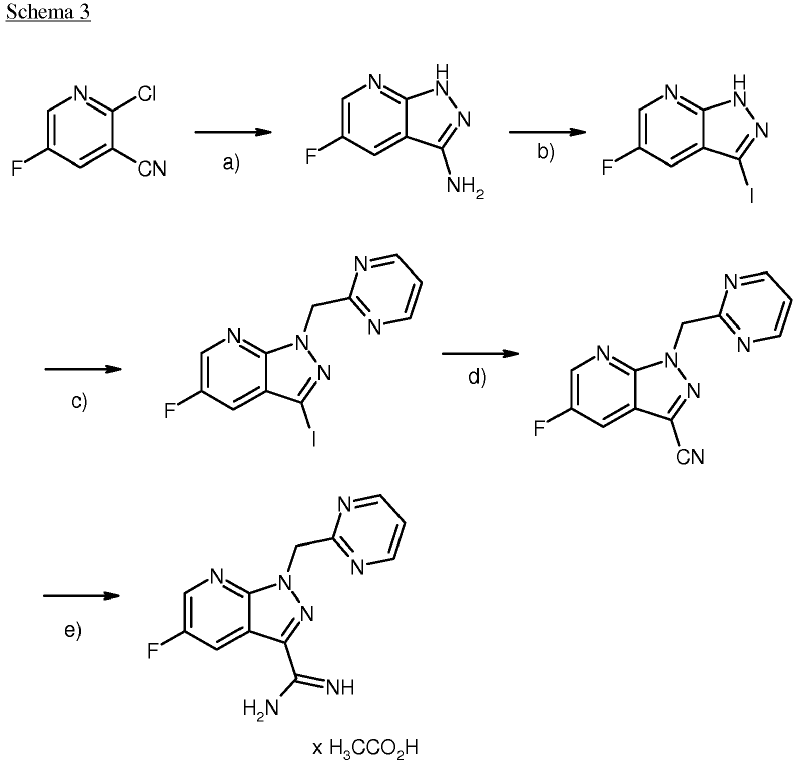 [a): Hydrazinhydrat, 1,2-Ethandiol; b): iso-Pentylnitrit, NaI, THF; b): 2-(Chlormethyl)pyrimidin, Cs₂CO₃, DMF; d): CuCN, DMSO, e): 1. NaOMe, MeOH, 2. NH₄Cl, Esssigsäure].

Alternativ erfolgt die Herstellung der Verbindungen der Formel (II) wie im nachfolgenden Syntheseschema (Schema 4) gezeigt: 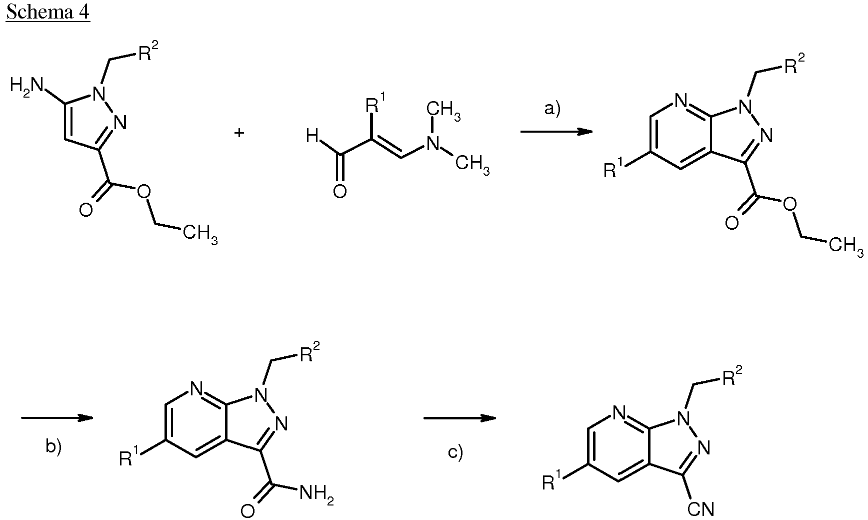 [a): TFA, Dioxan; b) NH₃; c) Trifluoressigsäureanhydrid].

Die Verbindung der Formel (XI) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 5) hergestellt werden: 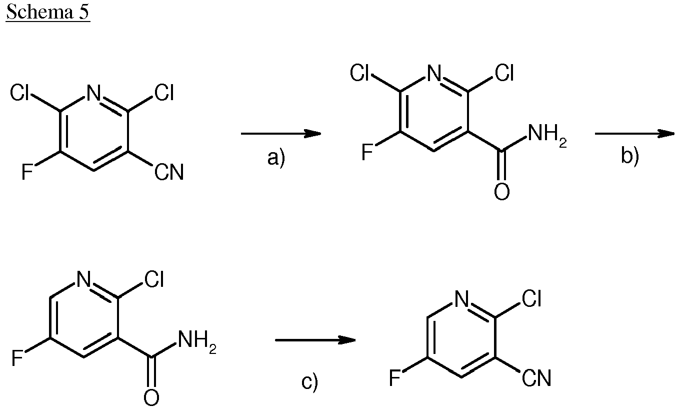 [a): Schwefelsäure; b): Zink, Methanol, Eisessig; c): Trifluoressigsäureanhydrid, Dichlormethan].

Die Verbindungen der Formeln (III) und (VII) sind kommerziell erhältlich, literaturbekannt oder können in Analogie zu literaturbekannten Verfahren hergestellt werden.

Die erfindungsgemäßen Verbindungen wirken als Stimulatoren der löslichen Guanylatcyclase und weisen ein gleiches oder verbessertes therapeutisches Profil gegenüber den aus dem Stand der Technik bekannten Verbindungen auf, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung oder sind Gegenstand der Offenbarung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: berechnet
- br s: breites Singulett (bei NMR)
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: gefunden
- h: Stunde(n)
- HPLC: Hochdruck-, Hochleistungsflüssigchromatographie
- HRMS: hochaufgelöste Massenspektrometrie
- konz.: konzentriert
- LC-MS: Flüssigchromatographie-gekoppelte Massenspektrometrie
- Me: Methyl
- min: Minute(n)
- MS: Massenspektrometrie
- NMR: Kernresonanzspektrometrie
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)

### LC/MS-Methoden:

### Methode 1:

Gerätetyp MS: Waters ZQ; Gerätetyp HPLC: Agilent 1100 Series; UV DAD; Säule: Thermo Hypersil GOLD 3 µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 2:

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.

### Methode3:

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 4:

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 5:

Instrument MS: Waters (Micromass) Quattro Micro; Instrument HPLC: Agilent 1100 Serie; Säule : YMC-Triart C183 µ 50 x 3 mm; Eluent A: 11 Wasser + 0.01 mol Ammoniumcarbonat, Eluent B: 1 1 Acetonitril; Gradient: 0.0 min 100% A → 2.75 min 5% A → 4.5 min 5% A; Ofen: 40°C; Fluss: 1.25 ml/min; UV-Detektion: 210 nm

### Ausgangsverbindungen und Intermediate:

### Beispiel 1A

### 2,6-Dichlor-5-fluornicotinamid

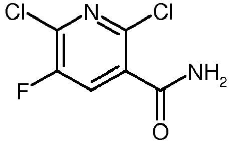

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.
Ausbeute: 24.5 g (90 % d. Theorie)
¹H-NMR (400 MHz, DMSO-d₆): δ = 7.95 (br s, 1H), 8.11 (br s, 1H), 8.24 (d, 1H).

### Beispiel 2A

### 2-Chlor-5-fluornicotinamid

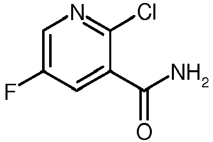

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min ausgerührt. Es wurde erneut abgesaugt und mit Diethylether nachgewaschen und trockengesaugt.
Ausbeute: 20.2 g (53 % d. Theorie)
¹H-NMR (400 MHz, DMSO-d₆): δ = 7.87 (br s, 1H), 7.99 (dd, 1H), 8.10 (br s, 1H), 8.52 (d, 1H).

### Beispiel 3A

### 2-Chlor-5-fluornicotinonitril

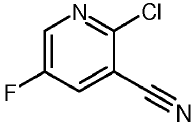

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.
Ausbeute: 42.1 g (90 % d. Theorie).
¹H-NMR (400 MHz, DMSO-d₆): δ = 8.66 (dd, 1H), 8.82 (d, 1H).

### Beispiel 4A

### 5-Fluor-1H-pyrazolo [3,4-b]pyridin-3-amin

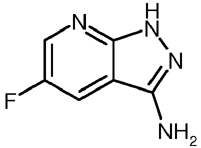

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml, 2.459 mol) versetzt. Es wurde unter Rühren für 4 h zum Rückfluss erhitzt. Beim Abkühlen fiel das Produkt aus. Die gelben Kristalle wurden mit Wasser (380 ml) versetzt und 10 min. bei RT ausgerührt. Anschliessend wurde die Suspension über eine Fritte abgesaugt, mit Wasser (200 ml) und mit -10°C-kaltem THF (200 ml) nachgewaschen. Der Rückstand wurde im Hochvakuum über Phosphorpentoxid getrocknet.
Ausbeute: 22.8 g (61 % d. Theorie)
¹H-NMR (400 MHz, DMSO-d₆): δ = 5.54 (s, 2H), 7.96 (dd, 1H), 8.38 (m, 1H), 12.07 (m, 1H).

### Beispiel 5A

### 5-Fluor-3-iod-1H-pyrazolo[3,4-b]pyridin

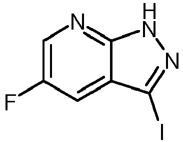

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.8 1) gegeben und zweimal mit Essigsäureethylester (je 487 ml) extrahiert. Die gesammelten organischen Phasen wurden mit gesättigter wässriger Natriumchlorid-Lösung (244 ml) gewaschen, getrocknet, filtriert und eingeengt. Man erhielt 12.1 g (86%-ige Reinheit, 60 % d. Th.) der gewünschten Verbindung als braunen Feststoff. Das Rohprodukt wurde ohne weitere Reinigung umgesetzt.
LC-MS (Methode 1): Rₜ = 1.68 min; MS (ESIpos): m/z = 264 (M+H)⁺

### Beispiel 6A

### 5-Fluor-3-iod-1-(pyrimidin-2-ylmethyl)-1H-pyrazolo [3,4-b]pyridin

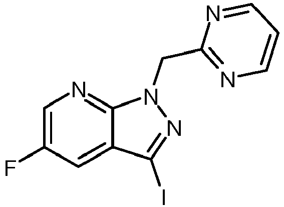

In DMF (50 ml) wurden 3.72 g (14.142 mmol) 5-Fluor-3-iod-1H-pyrazolo[3,4-b]pyridin und 5.069 g (15.557 mmol) Cäsiumcarbonat vorgelegt und anschließend 2.00 g (15.557 mmol) 2-(Chlormethyl)-pyrimidin in DMF (20 ml) gelöst zugetropft. Die Mischung wurde über Nacht bei RT gerührt. Anschließend wurde abgekühlt und auf 200 ml Wasser gegossen. Es bildete sich ein Niederschlag, der abfiltriert, mit Wasser gewaschen und über Nacht am Hochvakuum getrocknet wurde. Es wurden 2.26 g der Titelverbindung erhalten. Im Filtrat bildete sich anschliessend ein Niederschlag, der wiederum abfiltriert, mit Wasser gewaschen und am Hochvakuum über Nacht getrocknet wurde. Es wurden weitere 162 mg der Titelverbindung erhalten. Insgesamt erhielt man 2.42 g (48 % d. Th.) der Titelverbindung.
LC-MS (Methode 4): Rₜ = 1.85 min
MS (ESIpos): m/z = 356 (M+H)⁺
¹H-NMR (400 MHz, DMSO-d₆): δ = 5.91 (s, 2H), 7.43 (t, 1H), 7.95 (dd, 1H), 8.64 (m, 1H), 8.72 (d, 2H).

### Beispiel 7A

### 5-Fluor-1-(pyrimidin-2-ylmethyl)-1H-pyrazolo[3,4-b]pyridin-3-carbonitril

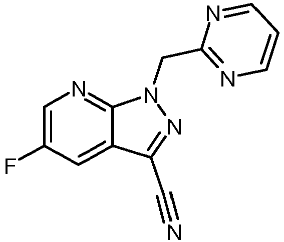

2.418 g (6.809 mmol) Beispiel 6A und 0.671 g (7.490 mmol) Kupfer-(I)-cyanid wurden in DMSO (40 ml) vorgelegt und 3 h bei 150°C gerührt. Nach Abkühlen wurde das Reaktionsgemisch mit gesättigter wässriger Ammoniumchloridlösung und konz. Ammoniakwasser (3:1 v/v) für 30 min verrührt, danach über Celite filtriert, und der Filterkuchen mit Essigsäureethylester nachgewaschen. Die Phasen des Filtrats wurden getrennt, und die organische Phase wurde danach dreimal mit einer Lösung aus gesättigter wässriger Ammoniumchloridlösung und konz. Ammoniakwasser (3:1 v/v) gewaschen. Nach Extraktion mit gesättigter wässriger Natriumchlorid-Lösung wurde über Natriumsulfat getrocknet, filtriert und im Vakuum eingeengt.
Ausbeute: 0.89 g (51 % d. Theorie)
LC-MS (Methode 4): Rₜ = 0.76 min
MS (ESIpos): m/z = 255 (M+H)⁺
¹H-NMR (400 MHz, DMSO-d₆): δ = 6.08 (s, 2H), 7.45 (t, 1H), 8.54 (dd, 1H), 8.72-8.74 (m, 2H), 8.80 (m, 1H).

### Beispiel 8A

### 5-Fluor-1-(pyrimidin-2-ylmethyl)-1H-pyrazolo[3,4-b]pyridin-3-carboximidamid-Acetat

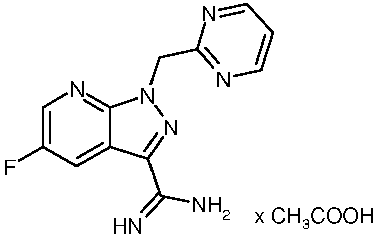

Zu 180 mg (3.343 mmol) Natriummethanolat in Methanol (5 ml) wurden 850 mg (3.343 mmol) Beispiel 7A in Methanol (2 ml) gegeben und 2 h bei RT gerührt. Danach wurden 214 mg (4.012 mmol) Ammoniumchlorid und Essigsäure (0.746 ml) zugegeben und über Nacht zum Rückfluss erhitzt. Danach wurde bis zur Trockene eingeengt und der Rückstand mit Essigsäureethylester und mit 1N Natronlauge versetzt. Die Phasen wurden getrennt. Die wässrige Phase wurde zweimal mit Essigsäureethylester extrahiert. Die wässrige Phase wurde eingeengt und der Rückstand mit DMF aufgeschlossen. Es wurde filtriert und der Filterkuchen mehrfach mit DMF nachgewaschen. Das Filtrat wurde anschliessend eingeengt und über Nacht im Hochvakuum getrocknet. Es konnten 514 mg (46 % d. Th.) der Titelverbindung erhalten werden.
LC-MS (Methode 2): Rₜ = 0.26 min
MS (ESIpos): m/z = 272 (M+H)⁺

### Beispiel 9A

### Methyl-3,3-dicyan-2,2-dimethylpropanoat

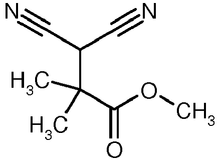

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben, und das Gemisch wurde über Nacht bei RT gerührt. Danach wurden nochmals 5.876 ml (45.411 mmol) Methyl-2-brom-2-methylpropanoat zugegeben und es wurde über Nacht auf 50°C erhitzt. Dann wurden abermals 1.762 ml (13.623) mmol) Methyl-2-brom-2-methylpropanoat zugegeben und es wurde weitere 4h auf 50°C erhitzt. Das Reaktionsgemisch wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, über Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.
Ausbeute: 6.47 g (85% d. Th.)
¹H-NMR (400 MHz, DMSO-d₆): δ [ppm] = 1.40 (s, 6H), 3.74 (s, 3H), 5.27 (s, 1H).

### Beispiel 10A

### 5-Fluor-1-(pyrimidin-2-ylmethyl)-1H-pyrazolo[3,4-b]pyridin-3-carboximidohydrazid

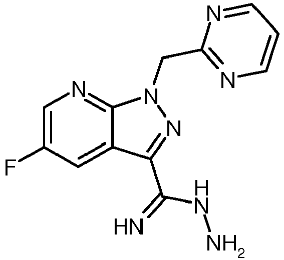

514 mg (1.551 mmol) der Verbindung aus Beispiel 8A wurden in 10.0 ml Ethanol gelöst und bei 0°C mit 627 mg (6.206mmol) Triethylamin sowie 97 mg (1.551 mmol) Hydrazinhydrat (80%-ige Lösung in Wasser) versetzt. Das Gemisch wurde über Nacht bei RT gerührt und anschließend am Rotationsverdampfer eingeengt. Der Rückstand wurde mit Essigsäureethylester und 10%iger wässriger Natriumchloridlösung extrahiert. Die Phasen wurden getrennt und die wässrige Phase wurde dreimal mit Essigsäureethylester extrahiert. Die vereinigten organischen Phasen wurden mit Natriumsulfat getrocknet, filtriert und bis zur Trockene eingeengt. Man erhielt 234 mg (52 % d. Th.) der Titelverbindung.
LC-MS (Methode 4): Rₜ = 0.72 min; MS (ESIpos): m/z = 287 (M+H)⁺

### Beispiel 11A

### Methyl-2-{3-[5-fluor-1-(pyrimidin-2-ylmethyl)-1H-pyrazolo[3,4-b]pyridin-3-yl]-5-hydroxy-1,2,4-triazin-6-yl} -2-methylpropanoat

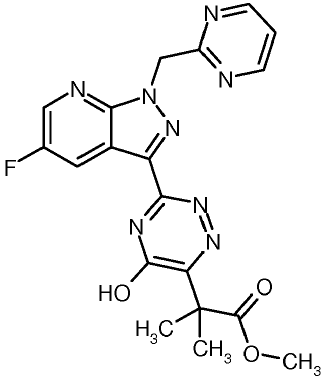

229 mg (1.221 mmol) Dimethyl-2,2-dimethyl-3-oxobutandioat (beschrieben in J. Am. Chem. Soc. 124(14), 3680-3691; 2002) wurden in 5 ml Ethanol vorgelegt und zum Rückfluss erhitzt. Danach wurden 233 mg (0.814 mmol) Beispiel 10A, suspendiert in 10 ml Ethanol, zugetropft. Das Gemisch wurde über Nacht zum Rückfluß erhitzt. Nach dem Abkühlen wurde das Reaktionsgemisch eingeengt und von einem Rückstand abfiltriert, welcher mit Ethanol nachgewaschen wurde. Das Filtrat wurde eingeengt und mit Diethylether behandelt. Es bildete sich ein Niederschlag, welcher abgetrennt und mit Diethylether nachgewaschen wurde. Man erhielt 164 mg der Titelverbindung (47 % d. Th.).
LC-MS (Methode 2): Rₜ = 0.76 min; MS (ESIpos): m/z = 425 (M+H)⁺
¹H-NMR (400 MHz, DMSO-d₆): δ [ppm] = 1.44 (s, 6H), 3.56 (s, 3H), 6.09 (s, 2H), 7.45 (t, 1H), 8.44 (dd, 1H), 8.74-8.77 (m, 3H), 14.60 (s br, 1H).

### Beispiel 12A

### 5-Fluor-1-[(3-fluorpyridin-2-yl)methyl]-3-iod-1H-pyrazolo[3,4-b]pyridin

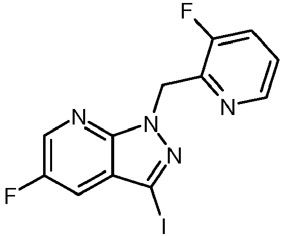

In DMF (10 ml) wurden 6.291 g (23.921 mmol) 5-Fluor-3-iod-1H-pyrazolo[3,4-b]pyridin und 8.573 g (26.313 mmol) Cäsiumcarbonat vorgelegt und anschließend 5.00 g (26.313 mmol) 2-(Brommethyl)-3-fluorpyridin in DMF (20 ml) gelöst zugetropft. Die Mischung wurde über Nacht bei RT gerührt. Anschließend wurde abgekühlt und auf 200 ml Wasser gegossen. Es wurde von einem Niederschlag abgesaugt, mit Wasser nachgewaschen und über Nacht im Hochvakuum getrocknet. Es wurden 6.28 g (70 % d. Th.) der Titelverbindung erhalten.
LC-MS (Methode 4): Rₜ = 2.17 min
MS (ESIpos): m/z = 373 (M+H)⁺
¹H-NMR (400 MHz, DMSO-d₆): δ = 5.88 (s, 2H), 7.42-7.46 (m, 1H), 7.77 (dd, 1H), 7.93 (dd, 1H), 8.27 (d,1H), 8.67 (t, 1H).

### Beispiel 13A

### 5-Fluor-1-[(3-fluorpyridin-2-yl)methyl]-1H-pyrazolo[3,4-b]pyridin-3-carbonitril

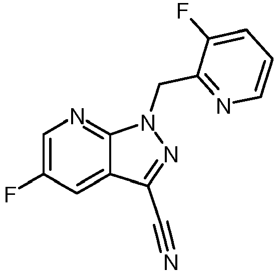

6.280 g (16.876 mmol) Beispiel 12A und 1.663 g (18.564 mmol) Kupfer-(I)-cyanid wurden in DMSO (100 ml) vorgelegt und 3 h bei 150°C gerührt. Nach Abkühlen wurde das Reaktionsgemisch über Celite filtriert und es wurde mit Essigsäureethylester nachgewaschen. Das Filtrat wurde viermal mit gesättigter wässriger Ammoniumchloridlösung und konz. Ammoniakwasser (3:1 v/v) extrahiert und die organische Phase abgetrennt. Diese wurde danach mit gesättigter wässriger Natriumchlorid-Lösung gewaschen, über Natriumsulfat getrocknet, filtriert und im Vakuum eingeengt. Es wurden 3.97 g (86 % d. Th.) der Titelverbindung erhalten.
LC-MS (Methode 2): Rₜ = 0.92 min
MS (ESIpos): m/z = 272 (M+H)⁺
¹H-NMR (400 MHz, DMSO-d₆): δ = 6.04 (s, 2H), 7.44-7.48 (m, 1H), 7.61 (t, 1H), 8.26 (d, 1H), 8.52 (dd, 1H), 8.83 (dd, 1H).

### Beispiel 14A

### 5-Fluor-1-[(3-fluorpyridin-2-yl)methyl]-1H-pyrazolo[3,4-b]pyridin-3-carboximidamid-Acetat

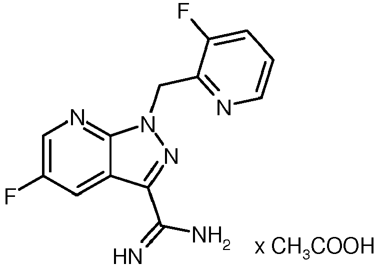

Zu 777 mg (14.379 mmol) Natriummethanolat in Methanol (20 ml) wurden 3.900 g (14.379 mmol) Beispiel 13A in Methanol (40 ml) gegeben und 2 h bei RT gerührt. Danach wurden 932 mg (17.255 mmol) Ammoniumchlorid und Essigsäure (3.210 ml) zugegeben und über Nacht zum Rückfluss erhitzt. Danach wurde das Reaktionsgemisch bis zur Trockene eingeengt und der Rückstand mit Essigsäureethylester und mit 1N Natronlauge versetzt und für 2h bei RT gerührt. Es wurde dann von einem Feststoff abfiltriert, welcher mit Essigsäureethylester und Wasser nachgewaschen wurde. Der Feststoff wurde über Nacht am Hochvakuum getrocknet. Es wurden 0.56 g (11 % d. Th.) der Titelverbindung erhalten. Die Phasen des Filtrats wurden getrennt, und die wässrige Phase wurde zweimal mit Essigsäureethylester extrahiert. Die vereinigten organischen Phasen wurden über Natriumsulfat getrocknet und eingeengt. Es wurden weitere 1.86 g (14 % d. Th., 39%ige Reinheit) der Titelverbindung erhalten. Die wässrige Phase wurde ebenfalls eingeengt, der Rückstand mit DMF versetzt und 30 min bei RT gerührt. Es wurde von einem Niederschlag abgesaugt, mit DMF nachgewaschen, das Filtrat eingeengt und über Nacht im Hochvakuum getrocknet. Es wurden weitere 1.77 g (35 % d. Th.) der Titelverbindung erhalten werden.
LC-MS (Methode 4): Rₜ = 1.25 min
MS (ESIpos): m/z = 289 (M+H)⁺

### Beispiel 15A

### 5-Fluor-1-[(3-fluorpyridin-2-yl)methyl]-1H-pyrazolo[3,4-b]pyridin-3-carboximidohydrazid

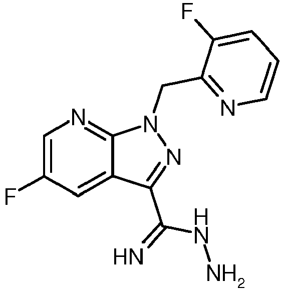

1.770 g (5.082 mmol) der Verbindung aus Beispiel 14A wurden in 40 ml Ethanol gelöst und bei 0°C mit 2.057 g (20.326 mmol) Triethylamin sowie 317 mg (5.082 mmol) Hydrazinhydrat (80%-ige Lösung in Wasser) versetzt. Das Gemisch wurde über Nacht bei RT gerührt und anschließend am Rotationsverdampfer eingeengt. Man erhielt 1.70 g der Titelverbindung als Rohprodukt, welches ohne weitere Reinigung in der nächsten Stufe umgesetzt wurde.
LC-MS (Methode 4): Rₜ = 1.23 min; MS (ESIpos): m/z = 304 (M+H)⁺

### Beispiel 16A

### Methyl-2-{3-[5-fluor-1-(pyrimidin-2-ylmethyl)-1H-pyrazolo[3,4-b]pyridin-3-yl]-5-hydroxy-1,2,4-triazin-6-yl} -2-methylpropanoat

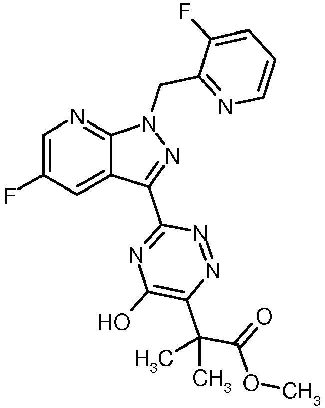

1.434 g (7.620 mmol) Dimethyl-2,2-dimethyl-3-oxobutandioat (beschrieben in J. Am. Chem. Soc. 124(14), 3680-3691; 2002) wurden in 30 ml Ethanol vorgelegt und zum Rückfluss erhitzt. Danach wurden 1.70 g der Rohverbindung aus Beispiel 15A, suspendiert in 30 ml Ethanol, zugetropft. Das Gemisch wurde über Nacht zum Rückfluß erhitzt. Nach dem Abkühlen wurde das Reaktionsgemisch eingeengt und von einem Rückstand abfiltriert. Das Filtrat wurde eingeengt und mit Diethylether versetzt. Es bildete sich ein Niederschlag, welcher abgetrennt und mit Diethylether nachgewaschen wurde. Das Filtrat wurde eingeengt und am Hochvakuum getrocknet. Dieser Rückstand wurde dann mit Diethylether versetzt, 10 min im Ultraschallbad behandelt und anschliessend 10 min bei RT verrührt. Es wurde ein Niederschlag abfiltriert, welcher mit wenig Diethylether nachgewaschen wurde und danach in Hochvakuum getrocknet wurde. Man erhielt so 356 mg der Titelverbindung (15 % d. Th.).
LC-MS (Methode 2): Rₜ = 0.93 min; MS (ESIpos): m/z = 442 (M+H)⁺
¹H-NMR (400 MHz, DMSO-d₆): δ [ppm] = 1.43 (s, 6H), 3.55 (s, 3H), 6.06 (s, 2H), 7.43-7.47 (m, 1H), 7.78-7.83 (m, 1H), 8.25 (d, 1H), 8.43 (dd, 1H), 8.78 (dd, 1H), 14.54 (br s, 1H).

### Beispiel 17A

### (3,5-Difluorpyridin-4-yl)methylmethansulfonat

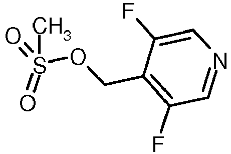

1.950 g (13.438 mmol) (3,5-Difluorpyridin-4-yl)methanol (beschrieben in WO 2010/132999) wurden in 50 ml Dichlormethan vorgelegt und bei 0°C mit 2.341 ml (13.438 mmol) N-Ethyl-N-isopropylpropan-2-amin versetzt. Anschliessend wurden 1.144 ml (14.782 mmol) Methansulfonsäurechlorid zugetropft und es wurde weitere 15 min bei 0°C gerührt und anschliessend über Nacht auf Raumtemperatur erwärmt. Danach wurde der Ansatz eingeengt und zweimal mit Toluol kodestilliert. Nach Trocknung im Hochvakuum erhielt man 5.39 g der Titelverbindung (ca. 15 % d. Th.), die ohne weitere Reinigung in der nächsten Stufe eingesetzt wurde.
LC-MS (Methode 2): Rₜ = 0.59 min; MS (ESIpos): m/z = 224 (M+H)⁺

### Beispiel 18A

### 1-[(3,5-Difluorpyridin-4-yl)methyl]-5-fluor-3-iod-1H-pyrazolo[3,4-b]pyridin

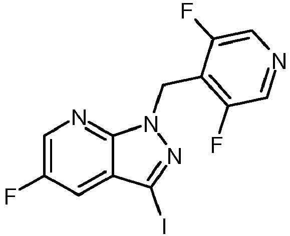

3.214 g (12.218 mmol) 5-Fluor-3-iod-1H-pyrazolo[3,4-b]pyridin wurden in Analogie zur Vorschrift unter Beispiel 12A mit Beispiel 17A umgesetzt. Es wurden 2.80 g (58 % d. Th.) der Titelverbindung erhalten.
LC-MS (Methode 2): Rₜ = 1.06 min
MS (ESIpos): m/z = 391 (M+H)⁺

### Beispiel 19A

### 1-[(3,5-Difluorpyridin-4-yl)methyl]-5-fluor-1H-pyrazolo[3,4-b]pyridin-3-carbonitril

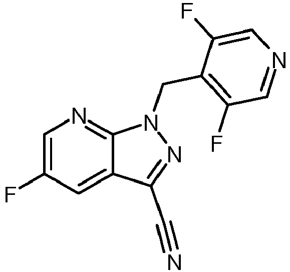

2.795 g (7.165 mmol) Beispiel 18A wurden in Analogie zur Vorschrift unter Beispiel 13A umgesetzt. Es wurden 1.95 g (93 % d. Th.) der Titelverbindung erhalten.
LC-MS (Methode 3): Rₜ = 0.95 min
MS (ESIpos): m/z = 290 (M+H)⁺

### Beispiel 20A

### 1-[(3,5-Difluorpyridin-4-yl)methyl]-5-fluor-1H-pyrazolo[3,4-b]pyridin-3-carboximidamid-Acetat

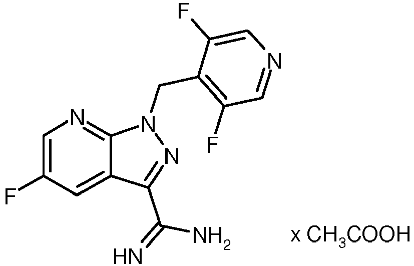

1.944 g (6.721 mmol) Beispiel 19A wurden in Analogie zur Vorschrift unter Beispiel 14A umgesetzt. Es wurden 1.96 g (79 % d. Th.) der Titelverbindung erhalten.
LC-MS (Methode 3): Rₜ = 0.44 min
MS (ESIpos): m/z = 307 (M+H)⁺

### Beispiel 21A

### 1-[(3,5-Difluorpyridin-4-yl)methyl]-5-fluor-1H-pyrazolo[3,4-b]pyridin-3-carboximidohydrazid

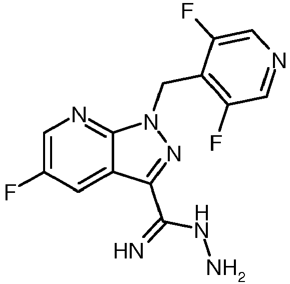

1.360 g (3.713 mmol) der Verbindung aus Beispiel 20A wurden in Analogie zur Vorschrift unter Beispiel 15A umgesetzt. Man erhielt 1.40 g der Titelverbindung als Rohprodukt, welches ohne weitere Reinigung in der nächsten Stufe umgesetzt wurde.
LC-MS (Methode 4): Rₜ = 1.30 min; MS (ESIpos): m/z = 322 (M+H)⁺

### Beispiel 22A

### Methyl-2-(3-{1-[(3,5-difluorpyridin-4-yl)methyl]-5-fluor-1H-pyrazolo[3,4-b]pyridin-3-yl}-5-hydroxy-1,2,4-triazin-6-yl)-2-methylpropanoat

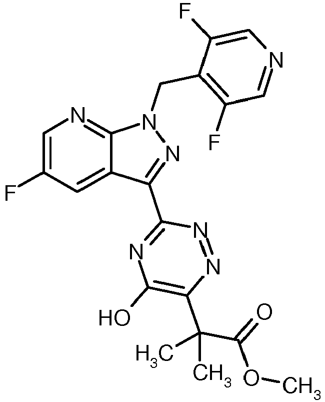

1.062 g (3.306 mmol) Beispiel 21A wurden in Analogie zur Vorschrift unter Beispiel 16A umgesetzt. Reinigung mittels präparativer HPLC (Acetonitril:Wasser-Gradient). Man erhielt so 240 mg der Titelverbindung (15 % d. Th.).
LC-MS (Methode 4): Rₜ = 2.01 min; MS (ESIpos): m/z = 460 (M+H)⁺

### Beispiel 23A

### 4-(Chlormethyl)-3-fluorpyridin-Hydrochlorid

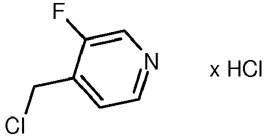

6.710 g (52.785 mmol) (3-Fluorpyridin-4-yl)methanol wurden in 29 ml Acetonitril vorgelegt und auf 50°C erhitzt. Danach wurde eine Lösung von 7.701 ml Thionylchlorid in 14.5 ml Acetonitril zugetropft und das Reaktionsgemisch wurde 4 h bei 50 °C gerührt. Danach wurde der Reaktionsansatz eingeengt und dreimal mit Dichlormethan kodestilliert. Nach Trocknung im Hochvakuum erhielt man 10.27 g der Titelverbindung, die ohne weitere Reinigung in der nächsten Stufe eingesetzt wurde.

### Beispiel 24A

### 5-Fluor-1-[(3-fluorpyridin-4-yl)methyl]-3-iod-1H-pyrazolo[3,4-b]pyridin

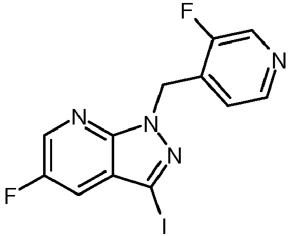

12.225 g (46.482 mmol) 5-Fluor-3-iod-1H-pyrazolo[3,4-b]pyridin wurden in Analogie zur Vorschrift unter Beispiel 12A mit Beispiel 23A umgesetzt. Es wurden 11.34 g (65 % d. Th.) der Titelverbindung erhalten.
LC-MS (Methode 3): Rₜ = 1.01 min
MS (ESIpos): m/z = 373 (M+H)⁺

### Beispiel 25A

### 5-Fluor-1-[(3-fluorpyridin-4-yl)methyl]-1H-pyrazolo[3,4-b]pyridin-3-carbonitril

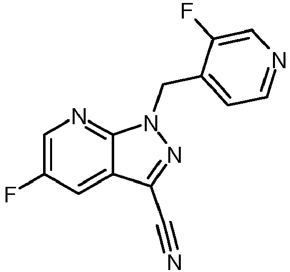

11.340 g (30.474 mmol) Beispiel 24A wurden in Analogie zur Vorschrift unter Beispiel 13A umgesetzt. Es wurden 6.31 g (76 % d. Th.) der Titelverbindung erhalten.
LC-MS (Methode 3): Rₜ = 0.89 min
MS (ESIpos): m/z = 272 (M+H)⁺

### Beispiel 26A

### 5-Fluor-1-[(3-fluorpyridin-4-yl)methyl]-1H-pyrazolo[3,4-b]pyridin-3-carboximidamid-Acetat

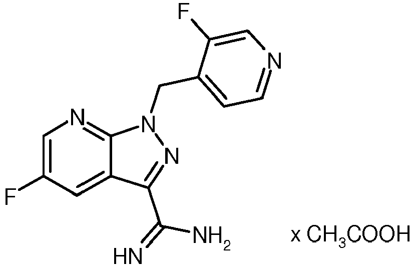

6.310 g (23.264 mmol) Beispiel 25A wurden in Analogie zur Vorschrift unter Beispiel 14A umgesetzt. Es wurden 6.12 g (75 % d. Th.) der Titelverbindung erhalten.
LC-MS (Methode 2): Rₜ = 0.45 min
MS (ESIpos): m/z = 289 (M+H)⁺

### Beispiel 27A

### 5-Fluor-1-[(3-fluorpyridin-4-yl)methyl]-1H-pyrazolo[3,4-b]pyridin-3-carboximidohydrazid

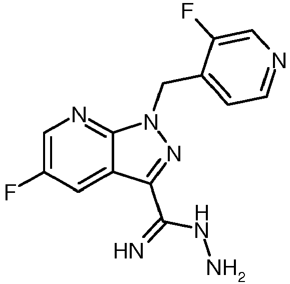

2.00 g (5.742 mmol) der Verbindung aus Beispiel 26A wurden in Analogie zur Vorschrift unter Beispiel 15A umgesetzt. Man erhielt 2.07 g der Titelverbindung als Rohprodukt, welches ohne weitere Reinigung in der nächsten Stufe umgesetzt wurde.
LC-MS (Methode 2): Rₜ = 0.37 min; MS (ESIpos): m/z = 304 (M+H)⁺

### Beispiel 28A

### Methyl-2-(3-{5-fluor-1-[(3-fluorpyridin-4-yl)methyl]-1H-pyrazolo[3,4-b]pyridin-3-yl}-5-hydroxy-1,2,4-triazin-6-yl)-2-methylpropanoat

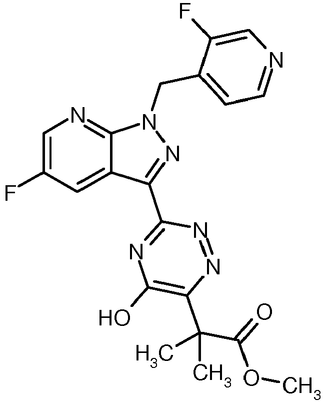

1.741 g (5.742 mmol) Beispiel 27A wurden in Analogie zur Vorschrift unter Beispiel 16A umgesetzt. Reinigung mittels präparativer HPLC (Acetonitril:Wasser-Gradient). Man erhielt so 740 mg der Titelverbindung (29 % d. Th.).
LC-MS (:Methode 2): Rₜ = 0.84 min; MS (ESIpos): m/z = 442 (M+H)⁺

### Beispiel 29A

### 5-Fluor-3-iod-1-(4-methoxybenzyl)-1H-pyrazolo[3,4-b]pyridin

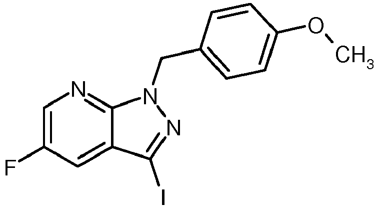

10.00 g (38.021 mmol) Beispiel 5A wurden in Analogie zur Vorschrift unter Beispiel 6A mit 4-Methoxybenzylchlorid umgesetzt. Man erhielt nach Chromatographie an Kieselgel (Eluent: Cyclohexan-Essigsäurethylester-Gemisch) 8.94 g (61 % d. Th.) der Titelverbindung.
LC-MS (Methode 3): Rₜ = 1.25 min
MS (ESIpos): m/z = 384 (M+H)⁺

### Beispiel 30A

### 5-Fluor-1-(4-methoxybenzyl)-1 H-pyrazolo [3,4-b]pyridin-3-carbonitril

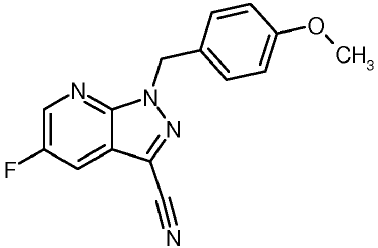

8.94 g (23.332 mmol) Beispiel 29A wurden in Analogie zur Vorschrift unter Beispiel 7A umgesetzt. Das so erhaltene Rohprodukt wurde ohne weitere Reinigung umgesetzt.
Ausbeute: 6.52 g (99 % d. Theorie)
LC-MS (Methode 2): Rₜ = 1.11 min
MS (ESIpos): m/z = 283 (M+H)⁺

### Beispiel 31A

### 5-Fluor-1-(4-methoxybenzyl)-1H-pyrazolo[3,4-b]pyridin-3-carboximidamid-Acetat

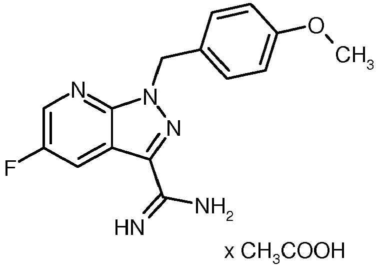

6.52 g (23.098 mmol) Beispiel 30A wurden in Analogie zur Vorschrift unter Beispiel 8A umgesetzt. Ausbeute: 6.16 g (74 % d. Theorie)
LC-MS (Methode 3): Rₜ = 0.55 min
MS (ESIpos): m/z = 300 (M+H)⁺

### Beispiel 32A

### 5-Fluor-1-(4-methoxybenzyl)-1H-pyrazolo[3,4-b]pyridin-3-carboximidohydrazid

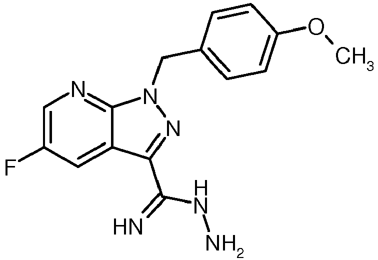

6.16 g (17.141 mmol) Beispiel 31 A wurden in Analogie zur Vorschrift unter Beispiel 15A umgesetzt. Eine Reinigung an Kieselgel wurde nicht durchgeführt. Es wurden 4.90 g (90 % d. Th.) der Titelverbindung erhalten.
LC-MS (Methode 3): Rₜ = 0.57 min; MS (ESIpos): m/z = 315 (M+H)⁺

### Beispiel 33A

### Methyl-2-{3-[5-fluor-1-(4-methoxybenzyl)-1H-pyrazolo[3,4-b]pyridin-3-yl]-5-hydroxy-1,2,4-triazin-6-yl}-2-methylpropanoat

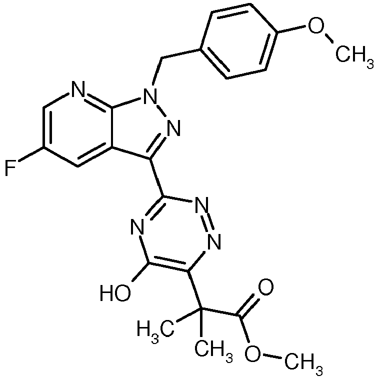

4.89 g (15.557 mmol) der Rohverbindung aus Beispiel 32A wurden in Analogie zur Vorschrift unter Beispiel 16A mit 4.391 g (23.336 mmol) Dimethyl-2,2-dimethyl-3-oxobutandioat (beschrieben in J. Am. Chem. Soc. 124(14), 3680-3691; 2002) umgesetzt. Nach vollständiger Umsetzung wurde ein Feststoff abfiltriert, welcher mit Ethanol nachgewaschen und anschliessend am Hochvakuum getrocknet wurde. Es wurden 6.04 g (85 % d. Th.) der Titelverbindung erhalten.
LC-MS (Methode 3): Rₜ = 1.05 min; MS (ESIpos): m/z = 453 (M+H)⁺

### Beispiel 34A

### 3-[5-Fluor-1-(4-methoxybenzyl)-1H-pyrazolo[3,4-b]pyridin-3-yl]-7,7-dimethyl-5,7-dihydro-6H-pyrrolo[2,3-e][1,2,4]triazin-6-on

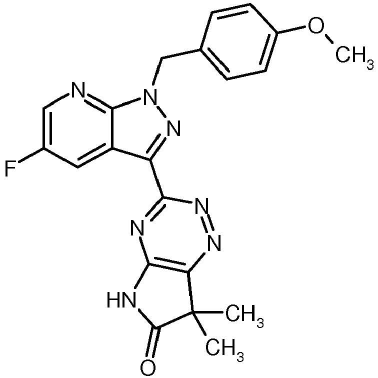

6.04 g (13.350 mmol) der Verbindung aus Beispiel 33A wurden in Analogie zur Vorschrift unter Beispiel 1 umgesetzt. Man erhielt nach Trocknung am Hochvakuum 1.27 g (22 % d. Th.) der Titelverbindung.
LC-MS (Methode 2): Rₜ = 1.02 min; MS (EIpos): m/z = 420 [M+H]⁺.
¹H-NMR (400 MHz, DMSO-d₆): δ [ppm] = 1.45 (s, 6H), 3.70 (s, 3H), 5.75 (s, 2H), 6.88 (d, 2H), 7.29 (d, 2H), 8.53 (dd, 1H), 8.78 (dd, 1H), 12.18 (s br, 1H).

### Beispiel 35A

### 3-[5-Fluor-1-(4-methoxybenzyl)-1H-pyrazolo[3,4-b]pyridin-3-yl]-7,7-dimethyl-5-{[2-(trimethylsilyl)ethoxy]methyl}-5,7-dihydro-6H-pyrrolo[2,3-e][1,2,4]triazin-6-on

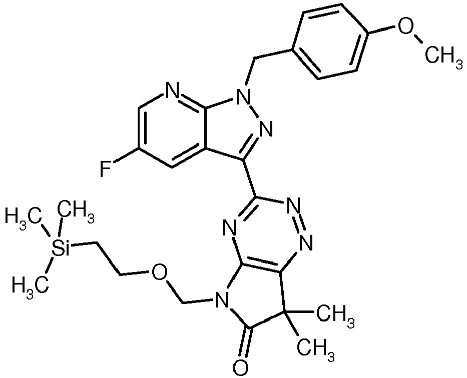

2.45 g (5.768 mmol) der Verbindung aus Beispiel 34A wurden mit 2.067 g (6.345 mmol) Cäsiumcarbonat in DMF (30 ml) versetzt. Danach wurden 1.221 ml (6.922 mmol) 2-(Trimethylsilyl)ethoxymethylchlorid zugegeben und es wurde 1 h bei Raumtemperatur gerührt. Anschliessend wurde von Feststoffen filtriert, mit DMF nachgewaschen, eingeengt und der Rückstand am Hochvakuum getrocknet. Es wurden 4.45 g Rohmaterial erhalten, die ohne weitere Reinigung in der nächsten Stufe eingesetzt wurden.
LC-MS (Methode 2): Rₜ = 1.43 min; MS (EIpos): m/z = 550 [M+H]⁺.

### Beispiel 36A

### 3-(5-Fluor-1H-pyrazolo[3,4-b]pyridin-3-yl)-7,7-dimethyl-5-{[2-(trimethylsilyl)ethoxy]methyl}-5,7-dihydro-6H-pyrrolo[2,3-e][1,2,4]triazin-6-on

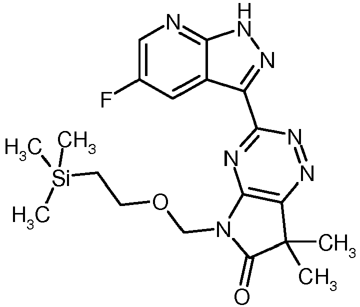

4.148 g (7.546 mmol) der Verbindung aus Beispiel 35A wurden in Acetonitril (110 ml) und Wasser (55 ml) aufgenommen und mit 12.411 g (22.638 mmol) Ammoniumcer(IV)-nitrat versetzt und für 20 min bei Raumtemperatur gerührt. Danach wurde mit viel Wasser versetzt und von einem Niederschlag filtriert. Dieser Feststoff wurde mit Wasser und anschliessend mit wenig Diethylether gewaschen. Es wurden 1.53 g (47 % d. Theorie) der Titelverbindung nach Trocknung am Hochvakuum erhalten.
LC-MS (Methode 2): Rₜ = 1.14 min; MS (EIpos): m/z = 430 [M+H]⁺.

### Beispiel 37A

### 3-{1-[(3,5-Difluorpyridin-2-yl)methyl]-5-fluor-1H-pyrazolo[3,4-b]pyridin-3-yl}-7,7-dimethyl-5-{[2-(trimethylsilyl)ethoxy]methyl}-5,7-dihydro-6H-pyrrolo[2,3-e][1,2,4]triazin-6-on

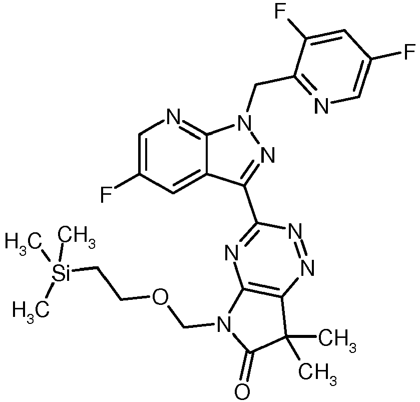

137 mg (0.524 mmol) Triphenylphosphin wurde in einem Kolben in 3 ml Tetrahydrofuran und 3 ml Dichlormethan gelöst und auf 0°C gekühlt. Danach wurden 101 µl (0.524 mmol) Diisopropylazodicarboxylat zugegeben und die Lösung wurde 1 h bei 0°C gerührt (Lösung 1). In einem weiteren Kolben wurden 0.150 g (0.349 mmol) der Verbindung aus Beispiel 36A und 76 mg (0.542 mmol) (3,5-Difluorpyridin-2-yl)methanol in Tetrahydrofuran (6 ml) gelöst und auf 0°C gekühlt (Lösung 2). Zu dieser Lösung 2 wurde anschliessend Lösung 1 hinzugegeben und das Reaktionsgemisch wurde 2 h bei Raumtemperatur gerührt. Anschliessend wurde nochmals Lösung 1 wie oben beschrieben aus 274 mg (1.048 mmol) Triphenylphosphin und 203 µl (1.048 mmol) Diisopropylazodicarboxylat hergestellt und zusammen mit 152 mg (1.048 mmol) (3,5-Difluorpyridin-2-yl)methanol bei 0°C zum Reaktionsgemisch gegeben. Nach 2 h bei Raumtemperatur wurde mittels präparativer HPLC gereinigt (Acetonitril:Wasser (+0.05 % Ameisensäure) - Gradient). Es wurden 64 mg der Titelverbindung als Gemisch von Isomeren (N1/N2-alkyliert, Verhältnis 4.5:1) erhalten (33% d. Th.).
LC-MS (Methode 2): Rₜ = 1.32 min (N2) und 1.36 min (N1); MS (EIpos): m/z = 557 [M+H]⁺.

### Beispiel 38A

### 5-Fluor-3-iod-1-(pyrimidin-5-ylmethyl)-1H-pyrazolo [3,4-b]pyridin

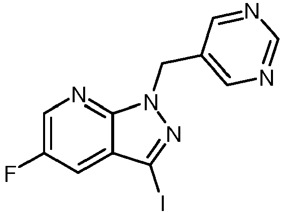

In 120 ml einer Mischung aus THF/Dichlormethan (v/v = 1:1) wurden 7.479 g (28.516 mmol) Triphenylphosphin gelöst und auf 0°C gekühlt. Zu der Lösung gab man 5.766 g (28.516 mmol) Diisopropylazodicarboxylat (DIAD) und ließ 1 h bei 0°C rühren. Parallel wurden 5.000 g (19.010 mmol) 5-Fluor-3-iod-1H-pyrazolo[3,4-b]pyridin und 3.140 g (28.516 mmol) Pyrimidin-5-ylmethanol (die Darstellung der Verbindung ist beschrieben in J. Org. Chem. 2000, 65, 9261) in 150 ml einer Mischung aus THF/Dichlormethan (v/v = 1:1) gelöst. Zu dieser Lösung addierte man tropfenweise die Triphenylphosphin/DIAD-Lösung bei 0°C. Man ließ auf RT erwärmen und rührte bei RT für 3 h. Die Reaktionsmischung wurde am Rotationsverdampfer eingeengt und mittels präparativer HPLC (Eluent: Wasser/Methanol/Wasser mit 1 % TFA, Verhältnis 60:35:5) gereinigt. Es wurden 3.730 g der Zielverbindung erhalten (Reinheit 93%, 51% d. Th.).
LC-MS (Methode 2): Rₜ = 0.80 min; MS (ESIpos): m/z = 356 (M+H)⁺

### Beispiel 39A

### 5-Fluor-1-(pyrimidin-5-ylmethyl)-1H-pyrazolo[3,4-b]pyridin-3-carbonitril

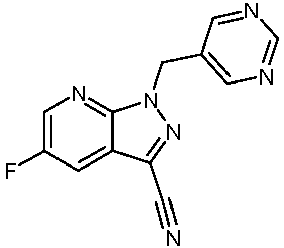

Unter Argonatmosphäre wurden 2.000 g (5.632 mmol) 1-5-Fluor-3-iod-1-(pyrimidin-5-ylmethyl)-1H-pyrazolo[3,4-b]pyridin und 555 mg (6.195 mmol) Kupfer(I)cyanid in 16 ml absolutem DMSO vorgelegt und 2 h bei 150°C erhitzt. Nach dem Abkühlen wurde der Ansatz über Celite filtriert und mit THF sowie Essigsäureethylester nachgewaschen. Die Lösung wurde zweimal mit einer Mischung aus 25%-iger Ammoniak-Lösung und gesättigter wässriger Ammoniumchlorid-Lösung (v/v = 1:3) gewaschen. Anschließend wurde die organische Phase mit einer gesättigten wässrigen Natriumchlorid-Lösung gewaschen, über Natriumsulfat getrocknet und am Rotationsverdampfer eingeengt. Der Rückstand wurde in Essigsäureethylester aufgenommen und über Kieselgel gesaugt und mit Essigsäureethylester nachgewaschen. Die organische Phase wurde eingeengt und der Rückstand im Hochvakuum getrocknet. Es wurden 931 mg (65 % d. Th.) der Zielverbindung erhalten.
LC-MS (Methode 4): Rₜ = 1.63 min; MS (ESIpos): m/z = 255 (M+H)⁺

### Beispiel 40A

### 5-Fluor-1-(pyrimidin-5-ylmethyl)-1H-pyrazolo[3,4-b]pyridin-3-carboximidamid-Acetat

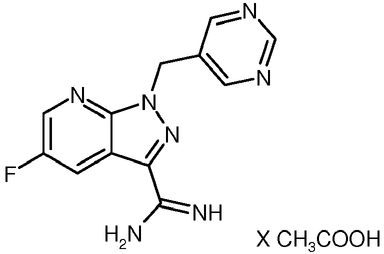

Unter Argonatmosphäre wurden 206 mg (3.664 mmol) Natriummethylat in 25 ml absolutem Methanol gelöst, mit 931 mg (3.664 mmol) 5-Fluor-1-(pyrimidin-5-ylmethyl)-1H-pyrazolo[3,4-b]pyridin-3-carbonitril versetzt und 1h bei RT gerührt. Es wurden 31 mg (0.550 mmol) Natriummethylat zugegeben und 15 Min. bei RT gerührt. Anschließend wurden 858 mg (14.288 mmol) Essigsäure sowie 482 mg (14.288 mmol) Ammoniumchlorid addiert und die Suspension für 1h zum Rückfluss erhitzt. Das Reaktionsgemisch wurde eingeengt und der Rückstand zwischen 1N Natronlauge und Essigsäureethylester verteilt. Die organische Phase wurden mit Natriumsulfat getrocknet und eingeengt. Es wurden 902 mg (86% d. Th.) der Zielverbindung erhalten.
LC-MS (Methode 5): Rₜ = 1.86 min; MS (ESIpos): m/z = 272 (M+H)⁺

### Beispiel 41A

### 5-Fluor-1-(pyrimidin-5-ylmethyl)-1H-pyrazolo[3,4-b]pyridin-3-carboximidohydrazid

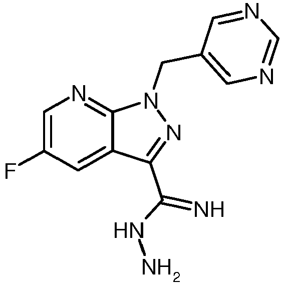

512 mg (1.700 mmol) 5-Fluor-1-(pyrimidin-5-ylmethyl)-1H-pyrazolo[3,4-b]pyridin-3-carboximid-amid wurden in 15 ml Ethanol vorgelegt und auf 0°C gekühlt. Es wurden 688 mg (6.800 mmol) Triethylamin sowie 106 mg (1.700 mmol) 80%-iges Hydrazinhydrat zugegeben und 18 h bei Raumtemperatur gerührt. Das Gemisch wurde am Rotationsverdampfer eingeengt und der Rückstand mit Essigsäureethylester verrührt. Das Filtrat wurde eingeengt, in Dichlormethan aufgenommen und mit 1 N Natronlauge sowie gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 88 mg (Reinheit 92%, 17% d. Th.) der Zielverbindung erhalten.
LC-MS (Methode 5): Rₜ = 1.78 min; MS (ESIpos): m/z = 287 (M+H)⁺

### Beispiel 42A

### Methyl-2-{3-[5-fluor-1-(pyrimidin-5-ylmethyl)-1H-pyrazolo[3,4-b]pyridin-3-yl]-5-hydroxy-1,2,4-triazin-6-yl}-2-methylpropanoat

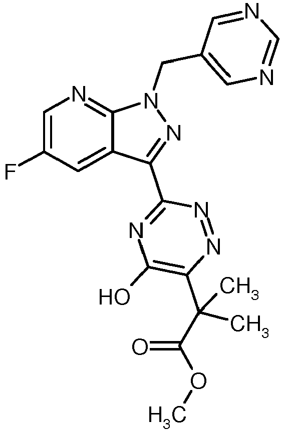

Es wurden 455 mg (2.418 mmol) Dimethyl-2,2-dimethyl-3-oxobutandioat in 10 ml Ethanol vorgelegt und zum Rückfluss erhitzt. Anschließend wurden 486 mg (1.612 mmol) 5-Fluor-1-(pyrimidin-5-ylmethyl)-1H-pyrazolo[3,4-b]pyridin-3-carboximidohydrazid suspendiert in 10 ml Ethanol addiert und über Nacht unter Rückfluss gekocht. Nach dem Abkühlen wurde das Gemisch eingeengt, mit Dichlormethan verrührt und filtriert. Das Filtrat wurde mittels präparativer HPLC (Eluent: Acetonitril/Wasser, Gradient 30:70 → 95:5) gereinigt. Es wurden 51 mg der Zielverbindung erhalten (7% d. Th.).
LC-MS (Methode 5): Rₜ = 1.75 min; MS (ESIpos): m/z = 425 (M+H)⁺

### Beispiel 43A

### (3-Fluor-2-thienyl)methanol

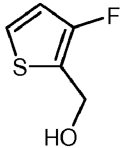

483 mg (3.016 mmol) 3-Fluorthiophen-2-carbonsäuremethylester wurden in 10 ml Tetrahydrofuran vorgelegt und bei 0°C mit 3.016 ml (3.016 mmol) Lithiumaluminiumhydrid (1 M in Tetrahydrofuran) versetzt Nach 1 h bei 0°C wurde über Nacht bei Raumtemperatur gerührt. Danach wurde der Ansatz langsam mit 1 ml Wasser, 1 ml Natronlauge (15 %ig) und 3 ml Wasser versetzt. Daraufhin wurde von einem Feststoff dekantiert und der Überstand wurde gesammelt. Der Feststoff wurde mit Tetrahydrofuran und Essigsäureethylester kurz im Ultraschallbad behandelt und anschliessend dekantiert. Die vereinigten Überstände wurden mit Wasser versetzt und anschliessend die Phasen getrennt. Die organische Phase wurde noch einmal mit gesättigter Natriumchloridlösung gewaschen und anschliessend mit Natriumsulfat getrocknet. Nach Filtration wurde im Vakuum eingeengt. Es wurden 417 mg der Zielverbindung erhalten (100% d. Th.), welche ohne weitere Reinigung im nächsten Schritt eingesetzt wurden.
¹H-NMR (400 MHz, DMSO-d₆): δ [ppm] = 4.54 (dd, 2H), 5.43 (t, 1H), 6.91 (dd, 1H), 7.44 (dd, 1H).

### Beispiel 44A

### 3-{5-Fluor-1-[(3-fluor-2-thienyl)methyl]-1H-pyrazolo[3,4-b]pyridin-3-yl}-7,7-dimethyl-5-{[2-(trimethylsilyl)ethoxy]methyl}-5,7-dihydro-6H-pyrrolo[2,3-e][1,2,4]triazin-6-on

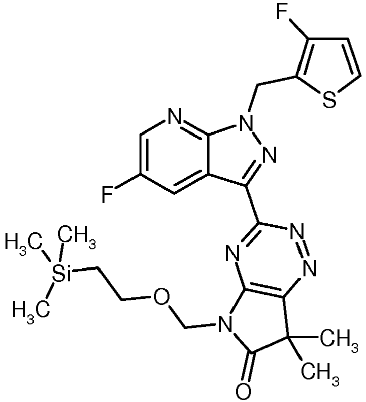

200 mg (0.466 mmol) der Verbindung aus Beispiel 36A wurden in Analogie zur Vorschrift unter Beispiel 37A mit 184 mg (1.397 mmol) Beispiel 43A umgesetzt. Nach 2 h bei Raumtemperatur wurde mittels präparativer HPLC gereinigt (Acetonitril:Wasser (+0.05 % Ameisensäure) - Gradient). Es wurden 58 mg der Titelverbindung als Gemisch von Isomeren (N1/N2-alkyliert, Verhältnis 4:1) erhalten (23% d. Th.).
LC-MS (Methode 2): Rₜ = 1.37 min (N2) und 1.42 min (N1); MS (EIpos): m/z = 544 [M+H]⁺.

### Ausführungsbeispiele

### Beispiel 1

### 3-[5-Fluor-1-(pyrimidin-2-ylmethyl)-1H-pyrazolo[3,4-b]pyridin-3-yl]-7,7-dimethyl-5,7-dihydro-6H-pyrrolo[2,3-e] [1,2,4]triazin-6-on

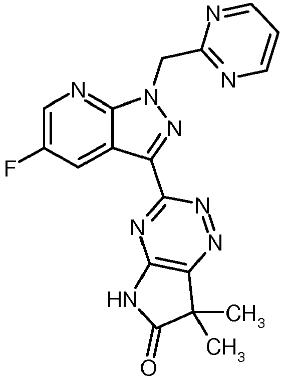

162 mg (0.382 mmol) der Verbindung aus Beispiel 11A wurden mit 2.600 ml Phosphorylchlorid versetzt und über Nacht bei RT gerührt. Das Reaktionsgemisch wurde in 25 ml Acetonitril gelöst und unter Eiskühlung in 16 ml konzentrierte wässriger Ammoniak-Lösung (33%-ig) eingerührt. Es wurde 3 Tage bei Raumtemperatur gerührt. Anschliessend wurde das Reaktionsgemisch eingeengt. Der Rückstand wurde mit Wasser und Essigsäureethylester extrahiert. Die Phasen wurden getrennt und die wässrige Phase wurde zweimal mit Essigsäureethylester extrahiert. Die vereinigten organischen Phasen wurden mit gesättigter wässriger Natriumchloridlösung gewaschen, über Natriumsulfat getrocknet, filtriert und eingeengt. Der Rückstand wurde mit Acetonitril versetzt. Es bildete sich ein Niederschlag, welcher abfiltriert, mit Acetonitril gewaschen und anschliessend im Hochvakuum getrocknet wurde. Es wurden 64 mg der Titelverbindung erhalten (43 % d. Th.)
LC-MS (Methode 3): Rₜ = 0.76 min; MS (EIpos): m/z = 392 [M+H]⁺.
¹H-NMR (400 MHz, DMSO-d₆): δ [ppm] = 1.46 (s, 6H), 6.06 (s, 2H), 7.44 (t, 1H), 8.58 (dd, 1H), 8.71-8.74 (m, 3H), 12.17 (br s, 1H).

### Beispiel 2

### 3-{5-Fluor-1-[(3-fluorpyridin-2-yl)methyl]-1H-pyrazolo[3,4-b]pyridin-3-yl}-7,7-dimethyl-5,7-dihydro-6H-pyrrolo[2,3-e][1,2,4]triazin-6-on

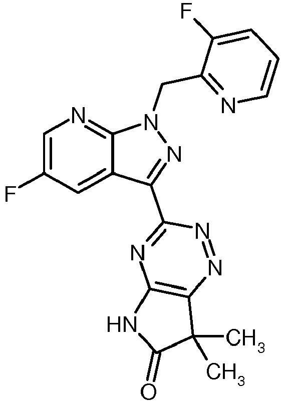

355 mg (0.804 mmol) der Verbindung aus Beispiel 16A wurden mit 5 ml Phosphorylchlorid versetzt und über Nacht bei RT gerührt. Das Reaktionsgemisch wurde in 53 ml Acetonitril gelöst und unter Eiskühlung in 34 ml konzentrierte wässriger Ammoniak-Lösung (33%-ig) eingerührt. Es wurde 3 Tage bei Raumtemperatur gerührt. Anschliessend wurde das Reaktionsgemisch eingeengt. Der Rückstand wurde mit Wasser und Ethanol versetzt und 1h bei RT gerührt. Es bildete sich ein Niederschlag, welcher abgesaugt, nacheinander mit Ethanol und Diethylether gewaschen und anschliessend im Hochvakuum getrocknet wurde. Es wurden 225 mg der Titelverbindung erhalten (66 % d. Th.)
LC-MS (Methode 3): Rₜ = 0.89 min; MS (EIpos): m/z = 409 [M+H]⁺.
¹H-NMR (400 MHz, DMSO-d₆): δ [ppm] = 1.45 (s, 6H), 6.03 (s, 2H), 7.42-7.47 (m, 1H), 7.76-7.81 (m, 1H), 8.27 (d, 1H), 8.55 (dd, 1H), 8.75 (dd, 1H), 12.19 (br s, 1H).

### Beispiel 3

### 4-Amino-2-{5-fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on

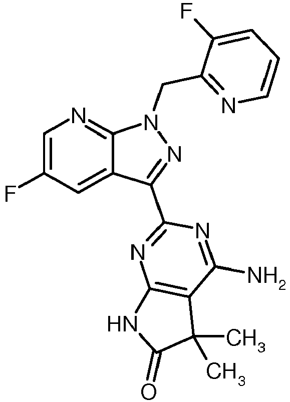

567 mg (1.628 mmol) Beispiel 14A wurden in tert.-Butanol (10 ml) vorgelegt und mit 274 mg (2.442 mmol) Kalium-tert.-butylat versetzt. Anschliessend wurden 324 mg (1.953 mmol) Beispiel 9A in tert.-Butanol (5 ml) zugegeben und die Mischung über Nacht zum Rückfluss erhitzt. Nach Abkühlen wurde das Reaktionsgemisch mit Wasser und Ethanol versetzt, und für 1 h gerührt. Der entstandene Niederschlag wurde abgesaugt und mit wenig Ethanol nachgewaschen. Der Feststoff wurde am Hochvakuum getrocknet. Es wurden 568 mg der Titelverbindung erhalten (80 % d. Th.).
LC-MS (Methode 3): Rₜ = 0.82 min; MS (ESIpos): m/z = 423 (M+H)⁺
¹H-NMR (400 MHz, DMSO-d₆): δ [ppm] = 1.34 (s, 6H), 5.94 (s, 2H), 6.87 (br s, 2H), 7.42-7.46 (m, 1H), 7.75-7.80 (m, 1H), 8.27 (d, 1H), 8.67 (dd, 1H), 8.83 (dd, 1H), 10.95 (br s, 1H).

### Gegenstand der Offenbarung ist Beispiel 4

### 2-{5-Fluor-1-[(3-fluorpyridin-2-yl)methyl]-1H-pyrazolo[3,4-b]pyridin-3-yl}-4-iod-5,5-dimethyl-5,7-dihydro-6H-pyrrolo[2,3-d]pyrimidin-6-on

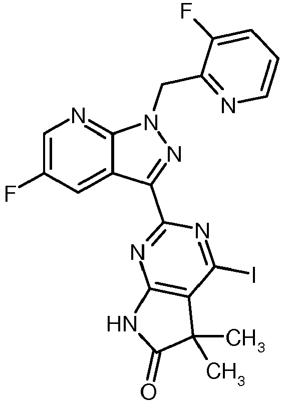

300 mg (0.710 mmol) von Beispiel 3 wurden in iso-Pentylnitrit (2.03 ml) und Diiodmethan (5.391 ml) vorgelegt und für 1h auf 85°C erhitzt. Nach Abkühlen wurde ein Feststoff abfiltriert, welcher mit wenig Acetonitril gewaschen wurde. Anschliessend wurde der Feststoff mittels präparativer HPLC gereinigt (Acetonitril:Wasser (+0.05 % Ameisensäure) - Gradient). Es wurden 58 mg der Titelverbindung erhalten (15% d. Th.).
LC-MS (Methode 4): Rₜ = 2.38 min; MS (ESIpos): m/z = 534 (M+H)⁺
¹H-NMR (400 MHz, DMSO-d₆): δ [ppm] = 1.42 (s, 6H), 6.02 (s, 2H), 7.42-7.46 (m, 1H), 7.76-7.81 (m, 1H), 8.26 (d, 1H), 8.48 (dd, 1H), 8.73 (dd, 1H), 11.75 (s, 1H).

Neben der Titelverbindung wurden ausserdem 25 mg (8% d.Th.) 2-{5-Fluor-1-[(3-fluorpyridin-2-yl)methyl]-1H-pyrazolo[3,4-b]pyridin-3-yl}-4-hydroxy-5,5-dimethyl-5,7-dihydro-6H-pyrrolo[2,3-d]pyrimidin-6-on (Beispiel 5) erhalten.

### Gegenstand der Offenbarung ist Beispiel 5

### 2-{5-Fluor-1-[(3-fluorpyridin-2-yl)methyl]-1H-pyrazolo[3,4-b]pyridin-3-yl}-4-hydroxy-5,5-dimethyl-5,7-dihydro-6H-pyrrolo [2,3-d]pyrimidin-6-on

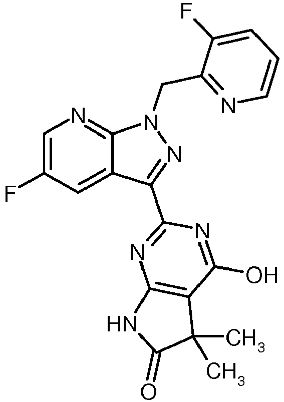

Die Titelverbindung entstand als Nebenkomponente in der Vorschrift unter Beispiel 4. Es wurden 25 mg (8% d.Th.) erhalten.
LC-MS (Methode 2): Rₜ = 0.83 min; MS (ESIpos): m/z = 424 (M+H)⁺
¹H-NMR (400 MHz, DMSO-d₆): δ [ppm] = 1.32 (s, 6H), 6.00 (s, 2H), 7.43-7.47 (m, 1H), 7.77-7.81 (m, 1H), 8.26 (d, 1H), 8.57 (br s, 1H), 8.75 (s, 1H), 11.09 (br s, 1H), 12.51 (br s, 1H).

### Beispiel 6

### 2-{5-Fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on

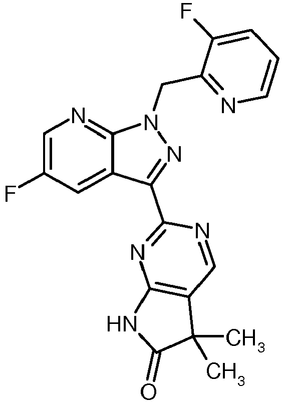

53 mg (0.099 mmol) von Beispiel 4 wurden in DMF (5 ml) gelöst und zu 23.88 mg Palladium auf Kohle (10 %) in DMF (1ml) gegeben und für 12 h bei Wasserstoff-Normaldruck hydriert. Anschliessend wurde über Celite filtriert, mit DMF nachgewaschen und bis zur Trockene eingeengt. Der Rückstand wurde mittels präparativer HPLC gereinigt (Acetonitril:Wasser (+0.05 % Ameisensäure) - Gradient). Es wurden 29 mg der Titelverbindung erhalten (71 % d. Th.).
LC-MS (Methode 2): Rₜ = 0.94 min; MS (ESIpos): m/z = 408 (M+H)⁺
¹H-NMR (400 MHz, DMSO-d₆): δ [ppm] = 1.38 (s, 6H), 5.99 (s, 2H), 7.42-7.47 (m, 1H), 7.76-7.81 (m, 1H), 8.27 (d, 1H), 8.59 (dd, 1H), 8.63 (s, 1H), 8.71 (dd, 1H), 11.56 (br s, 1H).

### Beispiel 7

### 4-Amino-2-{1-[(3,5-difluorpyridin-4-yl)methyl]-5-fluor-1H-pyrazolo[3,4-b]pyridin-3-yl}-5,5-dimethyl-5,7-dihydro-6H-pyrrolo[2,3-d]pyrimidin-6-on

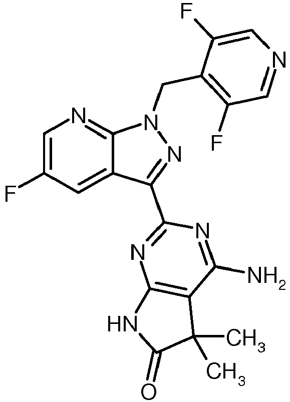

600 mg (1.638 mmol) Beispiel 20A wurden in Analogie zur Vorschrift unter Beispiel 3 umgesetzt. Es wurden 435 mg der Titelverbindung in ca. 59 %iger Reinheit erhalten. Hiervon wurde ein Teil mittels präparativer HPLC gereinigt (Acetonitril:Wasser (+0.05 % Ameisensäure) - Gradient). Es wurden 40 mg der Titelverbindung erhalten (5 % d. Th.).
LC-MS (Methode 2): Rₜ = 0.81 min; MS (ESIpos): m/z = 441 (M+H)⁺
¹H-NMR (400 MHz, DMSO-d₆): δ [ppm] = 1.33 (s, 6H), 5.91 (s, 2H), 6.88 (br s, 2H), 8.58 (s, 2H), 8.72 (m, 1H), 8.86 (dd, 1H), 10.99 (br s, 1H).

### Gegenstand der Offenbarung ist Beispiel 8

### 2-{1-[(3,5-Difluorpyridin-4-yl)methyl]-5-fluor-1H-pyrazolo[3,4-b]pyridin-3-yl}-4-iod-5,5-dimethyl-5,7-dihydro-6H-pyrrolo[2,3-d]pyrimidin-6-on

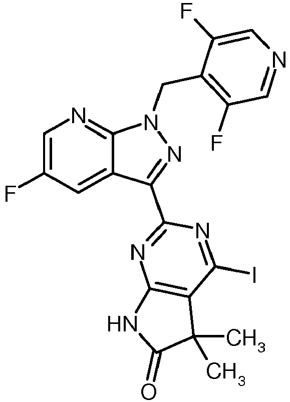

434 mg (0.985 mmol) Beispiel 7 wurden in Analogie zur Vorschrift unter Beispiel 4 umgesetzt. Es wurden 123 mg der Titelverbindung erhalten (22% d. Th.).
LC-MS (Methode 3): Rₜ = 1.15 min; MS (ESIpos): m/z = 552 (M+H)⁺
¹H-NMR (400 MHz, DMSO-d₆): δ [ppm] = 1.41 (s, 6H), 5.97 (s, 2H), 8.47 (dd, 1H), 8.58 (s, 2H), 8.79(dd, 1H), 11.77 (s, 1H).

Neben der Titelverbindung wurden ausserdem 13 mg (3% d.Th.) 2-{1[(3,5-Difluorpyridin-4-yl)methyl]-5-fluor-1H-pyrazolo[3,4-b]pyridin-3-yl}-4-hydroxy-5,5-dimethyl-5,7-dihydro-6H-pyrrolo[2,3-d]pyrimidin-6-on (Beispiel 9) erhalten.

### Gegenstand der Offenbarung ist Beispiel 9

### 2-{1-[(3,5-Difluorpyridin-4-yl)methyl]-5-fluor-1H-pyrazolo[3,4-b]pyridin-3-yl}-4-hydroxy-5,5-dimethyl-5,7-dihydro-6H-pyrrolo[2,3-d]pyrimidin-6-on

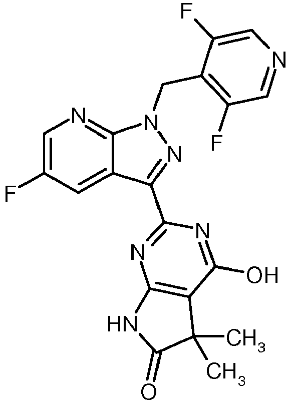

Die Titelverbindung entstand als Nebenkomponente in der Vorschrift unter Beispiel 8. Es wurden 13 mg (3% d.Th.) erhalten.
LC-MS (Methode 2): Rₜ = 0.83 min; MS (ESIpos): m/z = 442 (M+H)⁺
¹H-NMR (400 MHz, DMSO-d₆): δ [ppm] = 1.32 (s, 6H), 5.94 (s, 2H), 8.58 (s br, 3H), 8.81 (s br, 1H), 11.10 (s br, 1H), 12.53 (s br, 1H).

### Beispiel 10

### 2-{1-[(3,5-Difluorpyridin-4-yl)methyl]-5-fluor-1H-pyrazolo[3,4-b]pyridin-3-yl}-5,5-dimethyl-5,7-dihydro-6H-pyrrolo[2,3-d]pyrimidin-6-on

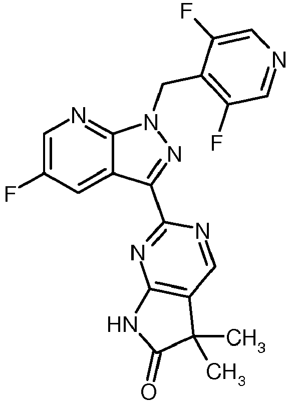

122 mg (0.221 mmol) von Beispiel 8 wurden in Analogie zur Vorschrift unter Beispiel 6 hydriert. Es wurden 72 mg der Titelverbindung erhalten (76 % d. Th.).
LC-MS (Methode 2): Rₜ = 0.96 min; MS (ESIpos): m/z = 426 (M+H)⁺
¹H-NMR (400 MHz, DMSO-d₆): δ [ppm] = 1.37 (s, 6H), 5.95 (s, 2H), 8.58-8.62 (m, 3H), 8.62 (s br, 1H), 8.77 (s br, 1H), 11.59 (br s, 1H).

### Beispiel 11

### 3-{1-[(3,5-Difluorpyridin-4-yl)methyl]-5-fluor-1H-pyrazolo[3,4-b]pyridin-3-yl}-7,7-dimethyl-5,7-dihydro-6H-pyrrolo[2,3-e][1,2,4]triazin-6-on

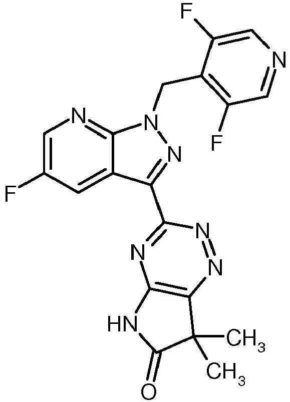

240 mg (0.522 mmol) der Verbindung aus Beispiel 22A wurden in Analogie zur Vorschrift unter Beispiel 2 umgesetzt. Es wurden 181 mg der Titelverbindung erhalten (80 % d. Th.)
LC-MS (Methode 2): Rₜ = 0.91 min; MS (EIpos): m/z = 427 [M+H]⁺.
¹H-NMR (400 MHz, DMSO-d₆): δ [ppm] = 1.44 (s, 6H), 5.98 (s, 2H), 8.55-8.59 (m, 3H), 8.81 (m, 1H), 12.19 (br s, 1H).

### Beispiel 12

### 4-Amino-2-{5-fluor-1-[(3-fluorpyridin-4-yl)methyl]-1H-pyrazolo[3,4-b]pyridin-3-yl}-5,5-dimethyl-5,7-dihydro-6H-pyrrolo[2,3-d]pyrimidin-6-on

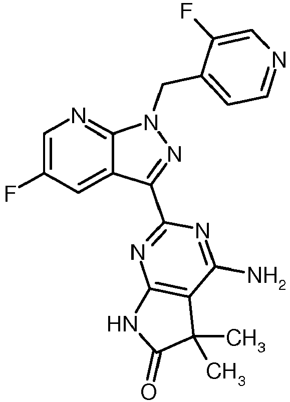

3.050 g (8.756 mmol) Beispiel 26A wurden in Analogie zur Vorschrift unter Beispiel 3 umgesetzt. Reinigung mittels präparativer Chromatographie an Kieselgel (Dichlormethan:Methanol - Gradient). Es wurden 528 mg der Titelverbindung erhalten (14 % d. Th,).
LC-MS (Methode 2): Rₜ = 0.80 min; MS (ESIpos): m/z = 423 (M+H)⁺
¹H-NMR (400 MHz, DMSO-d₆): δ [ppm] = 1.35 (s, 6H), 5.90 (s, 2H), 6.89 (br s, 2H), 7.11 (t, 1H), 8.35 (d, 1H), 8.59 (d, 1H), 8.70 (dd, 1H), 8.87 (dd, 1H), 10.99 (br s, 1H).

### Gegenstand der Offenbarung ist Beispiel 13

### 2-{5-Fluor-1-[(3-fluorpyridin-4-yl)methyl]-1H-pyrazolo[3,4-b]pyridin-3-yl}-4-iod-5,5-dimethyl-5,7-dihydro-6H-pyrrolo[2,3-d]pyrimidin-6-on

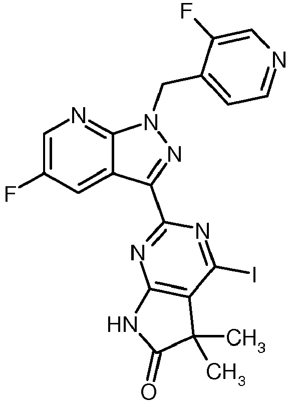

576 mg (1.364 mmol) Beispiel 12 wurden in Analogie zur Vorschrift unter Beispiel 4 umgesetzt. Es wurden 74 mg der Titelverbindung erhalten (10% d. Th.).
LC-MS (Methode 3): Rₜ = 1.10 min; MS (ESIpos): m/z = 534 (M+H)⁺
¹H-NMR (400 MHz, DMSO-d₆): δ [ppm] = 1.43 (s, 6H), 5.97 (s, 2H), 7.12 (t, 1H), 8.36 (d, 1H), 8.50 (dd, 1H), 8.60 (m, 1H), 8.78 (m, 1H), 11.77 (s, 1H).

### Beispiel 14

### 2-{s-Fluor-1-[(3-fluorpyridin-4-yl)methyl]-1H-pyrazolo[3,4-b]pyridin-3-yl}-5,5-dimethyl-5,7-dihydro-6H-pyrrolo[2,3-d]pyrimidin-6-on

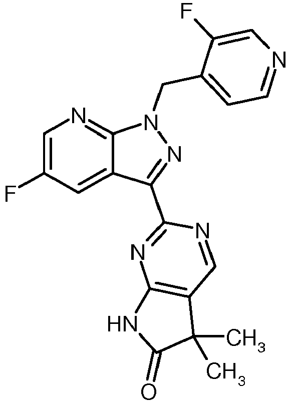

130 mg (0.244 mmol) von Beispiel 13 wurden in Analogie zur Vorschrift unter Beispiel 6 hydriert. Es wurden 54 mg der Titelverbindung erhalten (55 % d. Th.).
LC-MS (Methode 4): Rₜ = 1.99 min; MS (ESIpos): m/z = 408 (M+H)⁺
¹H-NMR (400 MHz, DMSO-d₆): δ [ppm] = 1.38 (s, 6H), 5.94 (s, 2H), 7.16 (t, 1H), 8.37 (d, 1H), 8.60-8.63 (m, 2H), 8.65 (s, 1H), 8.75 (dd, 1H), 11.60 (s, 1H).

### Beispiel 15

### 3-{5-Fluor-1-[(3-fluorpyridin-4-yl)methyl]-1H-pyrazolo[3,4-b]pyridin-3-yl}-7,7-dimethyl-5,7-dihydro-6H-pyrrolo[2,3-e][1,2,4]triazin-6-on

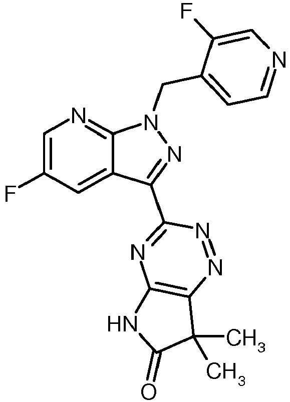

772 mg (1.680 mmol) der Verbindung aus Beispiel 28A wurden in Analogie zur Vorschrift unter Beispiel 2 umgesetzt. Es wurden 370 mg der Titelverbindung erhalten (53 % d. Th.).
LC-MS (Methode 4): Rₜ = 1.92 min; MS (EIpos): m/z = 409 [M+H]⁺.
¹H-NMR (400 MHz, DMSO-d₆): δ [ppm] = 1.45 (s, 6H), 5.98 (s, 2H), 7.18 (t, 1H), 8.37 (d, 1H), 8.57-8.60 (m, 2H), 8.79 (m, 1H), 12.17 (br s, 1H).

### Beispiel 16

### 3-{1-[(3,5-Difluorpyridin-2-yl)methyl]-5-fluor-1H-pyrazolo[3,4-b]pyridin-3-yl}-7,7-dimethyl-5,7-dihydro-6H-pyrrolo[2,3-e][1,2,4]triazin-6-on

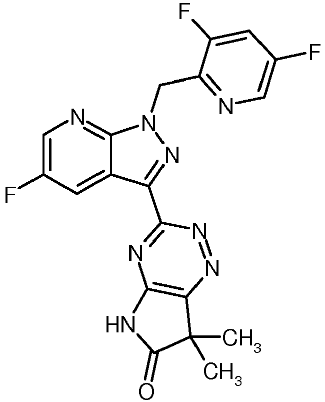

63 mg (0.114 mmol) der Verbindung aus Beispiel 37A wurden in Dichlormethan (4 ml) und Trifluoressigsäure (1 ml) 3 h bei Raumtemperatur gerührt. Anschliessend wurde bis zur Trockene eingeengt. Der Rückstand wurde in Ethanol/2N Salzsäure (4:1, 10 ml) 3 h bei 45 °C gerührt. Danach wurde bis zur Trockene eingeengt. Der erhaltene Rückstand wurde mittels präparativer HPLC gereinigt (Methanol:Wasser-Gradient). Nach Reinigung mittels präparativer HPLC (Methanol:Wasser - Gradient) wurden 21 mg der Titelverbindung erhalten (44% d. Th.).
LC-MS (Methode 2): Rₜ = 0.96 min; MS (EIpos): m/z = 427 [M+H]⁺.
¹H-NMR (400 MHz, DMSO-d₆): δ [ppm] = 1.45 (s, 6H), 6.01 (s, 2H), 8.00-8.05 (m, 1H), 8.38 (d, 1H), 8.55 (dd, 1H), 8.75 (dd, 1H), 12.16 (s br, 1H).

### Gegenstand der Offenbarung ist Beispiel 17

### 2-[5-Fluor-1-(pyrimidin-5-ylmethyl)-1H-pyrazolo[3,4-b]pyridin-3-yl]-4-iod-5,5-dimethyl-5,7-dihydro-6H-pyrrolo[2,3-d]pyrimidin-6-on

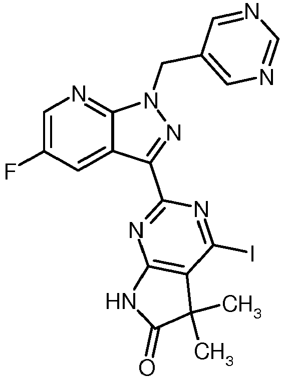

634 mg (1.563 mmol) 4-Amino-2-[5-fluor-1-(pyrimidin-5-ylmethyl)-1H-pyrazolo[3,4-b]pyridin-3-yl]-5,5-dimethyl-5,7-dihydro-6H-pyrrolo[2,3-d]pyrimidin-6-on wurden mit 35.234 g (131.551 mmol) Diiodmethan sowie 4.1 ml (30.449 mmol) Isopentylnitrit versetzt. Das Gemisch wurde 8 h bei 85 °C gerührt. Nach dem Abkühlen wurde filtriert, das Filtrat mit Cyclohexan verdünnt und über Kieselgel gesaugt. Das Kieselgel wurde mit Cyclohexan gewaschen und das Produkt mit Dichlormethan/Methanol (v/v = 100:3) euliert. Die gesammelten Fraktionen wurden am Rotationsverdampfer eingeengt und im Hochvakuum getrocknet. Es wurden 410 mg (Reinheit 68%, 34% d. Th.) der Zielverbindung erhalten.
LC-MS (Methode 3) Rₜ = 0.99 min; MS (ESIpos): m/z = 517 (M+H)⁺

### Beispiel 18

### 2-[5-Fluor-1-(pyrimidin-5-ylmethyl)-1H-pyrazolo[3,4-b]pyridin-3-yl]-5,5-dimethyl-5,7-dihydro-6H-pyrrolo[2,3-d]pyrimidin-6-on

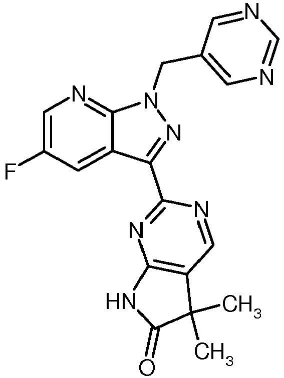

563 mg (Reinheit 65%, 0.709 mmol) 2-[5-Fluor-1-(pyrimidin-5-ylmethyl)-1H-pyrazolo[3,4-b]pyridin-3-yl]-4-iod-5,5-dimethyl-5,7-dihydro-6H-pyrrolo[2,3-d]pyrimidin-6-on wurden in 20 ml absolutem DMF gelöst, mit 150 mg 10% Palladium auf Kohle versetzt und unter Normaldruck mit Wasserstoff über Nacht hydriert. Das Reaktionsgemisch wurde über Celite filtriert und eingeengt. Der Rückstand wurde mittels päparativer HPLC gereinigt (Eluent: Acetonitril/Wasser, Gradient 30:70 → 95:5). Es wurden 47 mg (Reinheit 92%, 16% d. Th.) der Zielverbindung erhalten.
LC-MS (Methode 3) Rₜ = 0.78 min; MS (ESIpos): m/z = 391 (M+H)⁺
¹H-NMR (400MHz, DMSO-d₆): δ [ppm]= 1.38 (s, 6H), 5.90 (s, 2H), 8.60 (dd, 1H), 8.64 (s, 1H), 8.77 (dd, 1H), 8.64 (s, 2H), 9.15 (s, 1H).

### Beispiel 19

### 3-[5-Fluor-1-(pyrimidin-5-ylmethyl)-1H-pyrazolo[3,4-b]pyridin-3-yl]-7,7-dimethyl-5,7-dihydro-6H-pyrrolo[2,3-e] [1,2,4]triazin-6-on

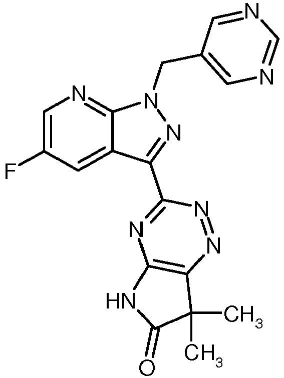

Es wurden 305 mg (Reinheit 62%, 0.445 mmol) Methyl-2-{3-[5-fluor-1-(pyrimidin-5-ylmethyl)-1H-pyrazolo[3,4-b]pyridin-3-yl]-5-hydroxy-1,2,4-triazin-6-yl}-2-methylpropanoat mit 5 ml (53.642 mmol) Phosphorylchlorid versetzt und über Nacht bei RT gerührt. Die Reaktionslösung wurde mit 20 ml trockenem Acetonitril verdünnt, unter Eiskühlung langsam in 112 ml einer 25%-igen wässrigen Ammoniak-Lösung getropft und über Nacht bei RT gerührt. Das Reaktionsgemisch wurde am Rotationsverdampfer eingeengt, bis das Acetonitril abgedampft war, und der Niederschlag abfiltriert und im Hochvakuum getrocknet. Es wurden 194 mg der Zielverbindung erhalten (97% d. Th.).
LC-MS (Methode 2) Rₜ = 0.79 min; MS (ESIpos): m/z = 392 (M+H)⁺
¹H-NMR (400MHz, DMSO-d₆): δ [ppm]= 1.30 (s, 6H), 5.91 (s, 2H), 8.56 (dd, 1H), 8.76 (dd, 1H), 8.84 (s, 2H), 9.14 (s, 1H).

### Beispiel 20

### 3-{5-Fluor-1-[(3-fluor-2-thienyl)methyl]-1H-pyrazolo[3,4-b]pyridin-3-yl}-7,7-dimethyl-5,7-dihydro-6H-pyrrolo[2,3-e][1,2,4]triazin-6-on

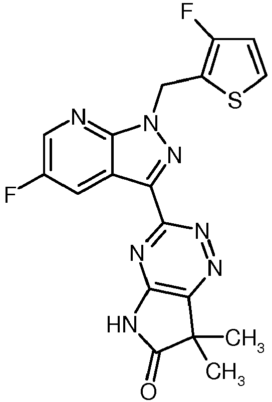

57.7 mg (0.106 mmol) der Verbindung aus Beispiel 44A wurden Analogie zur Vorschrift unter Beispiel 16 umgesetzt. Nach Reinigung mittels präparativer HPLC (Acetonitril: Wasser: Wasser mit 1% Trifluoressigsäure - (50:40:10) wurden 16 mg der Titelverbindung erhalten (37% d. Th.).
LC-MS (Methode 2): Rₜ = 1.02 min; MS (EIpos): m/z = 414 [M+H]⁺.
¹H-NMR (400 MHz, DMSO-d₆): δ [ppm] = 1.46 (s, 6H), 5.93 (s, 2H), 6.98 (d, 1H), 7.50 (t, 1H), 8.54 (dd, 1H), 8.81 (s br, 1H), 12.20 (s br, 1H).

### Beispiel 21

### 4-Amino-2-[5-fluor-1-(pyrimidin-5-ylmethyl)-1H-pyrazolo[3,4-b]pyridin-3-yl]-5,5-dimethyl-5,7-dihydro-6H-pyrrolo[2,3-d]pyrimidin-6-on

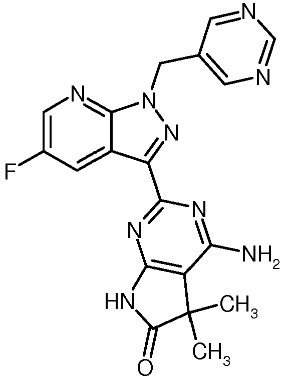

802 mg (2.803 mmol) 5-Fluor-1-(pyrimidin-5-ylmethyl)-1H-pyrazolo[3,4-b]pyridin-3-carboximidamid wurden mit 6 ml *tert*.-Butanol, 582 mg (2.803 mmol) Methyl-3,3-dicyan-2,2-dimethylpropanoat gelöst in 3 ml *tert*.-Butanol sowie 377 mg (3.363 mmol) Kalium-*tert*.-butylat versetzt und 2 h unter Rückfluss erhitzt. Nach dem Abkühlen wurde das Reaktionsgemisch filtriert und das Filtrat am Rotationsverdampfer eingeengt. Der Rückstand wurde mit Wasser/Acetoniril und dann mit Dichlormethan verrührt, abgesaugt und im Hochvakuum getrocknet. Es wurden 263 mg (23% d. Th.) der Zielverbindung erhalten.
LC-MS (Methode 4) Rₜ = 1.65 min; MS (ESIpos): m/z = 406 (M+H)⁺

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] | Beispiel Nr. | IC₅₀ [nM] | Beispiel Nr. | IC₅₀ [nM] |
|---|---|---|---|---|---|
| 1 | 1320 | 7 | 116 | 15 | 2230 |
| 2 | 234 | 10 | 259 | 18 | 196 |
| 3 | 34 | 11 | 2540 | 19 | 361 |
| 5 | 627 | 12 | 121 | 21 | 99 |
| 6 | 82 | 14 | 207 | | |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] | Beispiel Nr. | MEC [µM] |
|---|---|---|---|
| 1 | 10 | 11 | 3.0 |
| 2 | 1 | 12 | 1.0 |
| 3 | 0.3 | 14 | 1.0 |
| 4 | 0.3 | 15 | 3.0 |
| 5 | 3 | 16 | 1.0 |
| 6 | 0.03 | 18 | 0.1 |
| 7 | 0.3 | 19 | 0.1 |
| 9 | 3.0 | 20 | 0.01 |
| 10 | 1.0 | 21 | 0.1 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen der Formel (I) werden in männlichen CD-1-Mäusen, männlichen Wister-Ratte und/oder weiblichen Beagle-Hunden bestimmt. Das Applikationsvolumen beträgt bei Mäusen 5 mL/kg, bei Ratten 5 mL/kg und bei Hunden 0.5 mL/kg. Die intravenöse Gabe erfolgt bei Mäusen und Ratten mittels einer speziesspezifischen Plasma/DMSO-Formulierung (99/1) und bei Hunden mittels Wasser/PEG400/Ethanol (50/40/10 bzw. 30/60/10). Den Ratten wird zur vereinfachten Blutabnahme vor der Substanzgabe ein Silikonkatheter in die rechte *Vena jugularis externa* gelegt. Die Operation erfolgt einen Tag vor dem Versuch unter Isofluran-Narkose und unter Gabe eines Analgetikums (Atropin/Rimadyl (3/1) 0.1 mL s.c.). Die Substanzgabe wird bei Mäusen i.v. bolus, bei Ratten i.v. bolus oder mittels 15-minütiger Infusion und Hunden mittels 15-minütiger Infusion durchgeführt. Die Blutabnahme erfolgt bei Mäusen nach 0.033, 0.083, 0.17, 0.5, 1, 2, 3, 4, 6, 7 und 24 Stunden sowie bei Hunden und Ratten bei 15- minütiger Infusion nach 0.083, 0.25, 0.28 0.33, 0.42, 0.75, 1, 2, 3, 4, 6, 7 (bzw. 8 für Beispiel 3) und 24 Stunden sowie bei Ratten nach i.v. bolus-Applikation nach 0.033, 0.083, 0.17, 0.5, 1, 2, 3, 4, 6, 7 und 24 Stunden. Die orale Gabe der gelösten Substanz mittels Schlundsonde wird in allen Spezies basierend auf einer Wasser/PEG400/Ethanol-Formulierung (50/40/10) durchgeführt. Die Blutabnahme in Ratten und Hunden erfolgt hier nach 0.083, 0.17, 0.5, 0.75, 1, 2, 3, 4, 6, 7 und 24 Stunden. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen der Formel (I), Kalibrierproben und QC's wird ein interner Standard zugesetzt (ZK 228859) und es folgt eine Proteinfällung mittels Acetonitril im Überschuss. Nach Zugabe eines Ammoniumacetat-Puffers (0.01 M, pH 6.8) und folgendem Vortexen wird bei 1000 g zentrifugiert und der Überstand mittels LC-MS/MS (API 4000, AB Sciex) vermessen. Die chromatographische Trennung wird auf einer 1100-HPLC von Agilent durchgeführt. Das Injektionsvolumen beträgt 10 µL. Als Trennsäule wird eine auf 40°C temperierte Luna 5µ C8(2) 100A 50x2mm der Fa. Phenomenex verwendet. Es wird ein binärer Laufmittelgradient bei 500 µL/min gefahren (A: 0.01M Ammoniumacetat-Puffer pH 6.8, B: 0.1 % Ameisensäure in Acetonitril): 0 Min. (90 % A), 1 Min. (90 % A), 3 Min. (10 % A), 4 Min. (10 % A), 4.50 Min. (90 % A), 6 Min. (90 % A). Die Temperatur der Turbo V Ionenquelle beträgt 500°C (Beispiel 2), 400°C (Beispiel 3) bzw. 450°C (Beispiel 6). Folgende MS-Geräteparameter werden benutzt: Curtain Gas 15 units (Beispiel 2 und 3) bzw. 10 units (Beispiel 6), Ionsprayvoltage 4.8 kV, Gas 1 45 units (Beispiel 2) bzw. 50 units (Beispiel 3 und 6), Gas 2 35 units (Beispiel 2 und 6) bzw. 40 units (Beispiel 3), CAD Gas 4 units (Beispiel 2 und 6) bzw. 8 units (Beispiel 3). Die Quantifizierung der Substanzen erfolgt anhand der Peakhöhen oder -flächen aus extrahierten Ionenchromatogrammen spezifischer MRM-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels des validierten pharmakokinetischen Rechenprogramms KinEx (Vers. 3) berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (s.o.) und durch Quotientenbildung der C_{Blut}/C_{Plasma}-Wert ermittelt.

Tabelle 3 zeigt Daten repräsentativer Verbindungen der vorliegenden Erfindung nach intravenöser Gabe von 0.3 mg/kg in Ratten:

**Tabelle 3:**

| Beispiel | 2 | 3 | 6 |
|---|---|---|---|
| AUC ₙₒᵣₘ [kg·h/L] | 13 | 0.93 | 3.5 |
| CL_{Blut} [L/h/kg] | 0.13 | 0.93 | 0.37 |
| MRT [h] | 7.9 | 1.3 | 5.6 |
| t_{1/2} [h] | 6.1 | 1.0 | 4.4 |

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindungen der Formel (I-1), in welcher 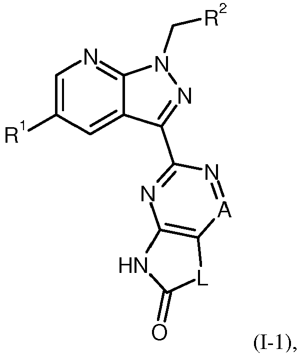 in welcher
A für Stickstoff oder CR³ steht,
wobei
R³ für Wasserstoff oder Amino steht,
L für eine Gruppe *-CR^{4A}R^{4B}-(CR^{5A}R^{5B})ₚ-# steht,
wobei
* für die Anknüpfstelle an die Carbonylgruppe steht,
# für die Anknüpfstelle an den Pyrimidinring bzw Triazinringsteht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
R¹ für Wasserstoff oder Fluor steht,
R² für Thienyl, Pyridyl oder Pyrimidinyl steht,
wobei Thienyl, Pyridyl und Pyrimidinyl mit 1 oder 2 Substituenten Fluor substituiert sein können,
sowie ihre Salze, Solvate und Solvate der Salze.

2. Verfahren zur Herstellung von Verbindungen der Formel (I), wie in Anspruch 1 definiert, **dadurch gekennzeichnet, dass** man die Verbindung der Formel (II) 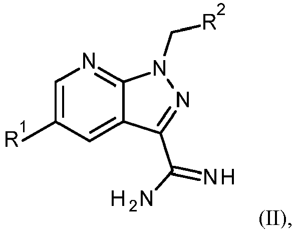 in welcher R¹ und R² jeweils die in Anspruch 1 genannten Bedeutungen haben,
[A] in einem inerten Lösungsmittel in Gegenwart einer geeigneten Base mit einer Verbindung der Formel (III) 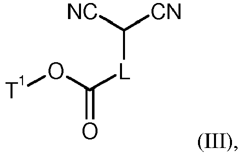
in welcher L die in Anspruch 1 genannte Bedeutung hat und
T¹ für (C₁-C₄)-Alkyl steht,
zu einer Verbindung der Formel (IV) 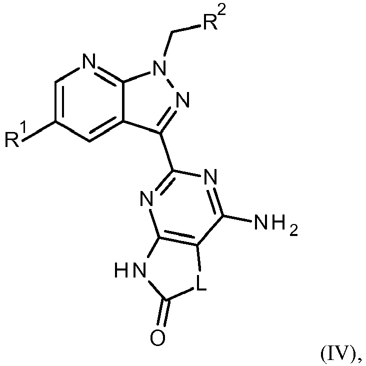
in welcher L, R¹ und R² jeweils die in Anspruch 1 angegebenen Bedeutungen haben,
umsetzt, diese dann mit iso-Pentylnitrit und einem Halogen-Äquivalent in eine Verbindung der Formel (V) 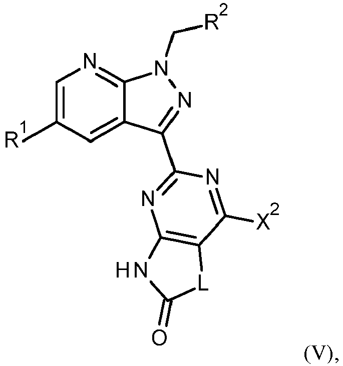
in welcher L, R¹ und R² jeweils die in Anspruch 1 angegebenen Bedeutungen haben und
X² für Brom oder Iod steht,
überführt, und diese im Anschluss in einen inerten Lösungsmittel in Gegenwart eines geeigneten Übergangsmetallkatalysators zu einer Verbindung der Formel (I-A) 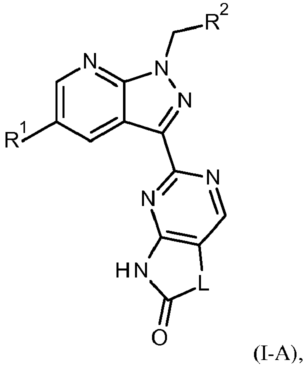
in welcher L, R¹ und R² jeweils die in Anspruch 1 angegebenen Bedeutungen haben,
umsetzt,
oder
[B] in einem inerten Lösungsmittel in Gegenwart einer geeigneten Base mit Hydrazinhydrat zu einer Verbindung der Formel (VI) 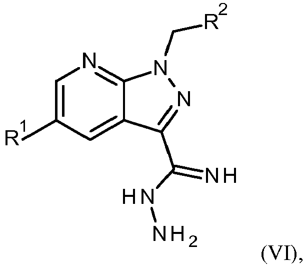
in welcher R¹ und R² jeweils die in Anspruch 1 genannten Bedeutungen haben,
umsetzt, diese dann in einem inerten Lösungsmittel mit einer Verbindung der Formel (VII) 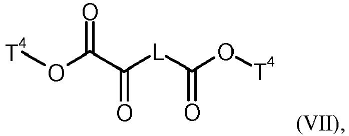
in welcher L die in Anspruch 1 angegebene Bedeutung hat und
T⁴ für (C₁-C₄)-Alkyl steht,
zu einer Verbindung der Formel (VIII) 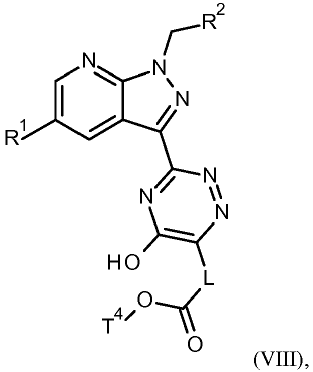
in welcher L, R¹ und R² und T⁴ jeweils die in Anspruch 1 angegebenen Bedeutungen haben,
reagiert, diese anschliessend mit Phosphorylchlorid in eine Verbindung der Formel (IX) 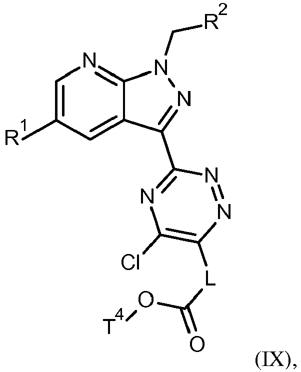
in welcher L, R¹ und R² und T⁴ jeweils die in Anspruch 1 angegebenen Bedeutungen haben,
überführt, und diese direkt mit Ammoniak zu einer Verbindung der Formel (X) 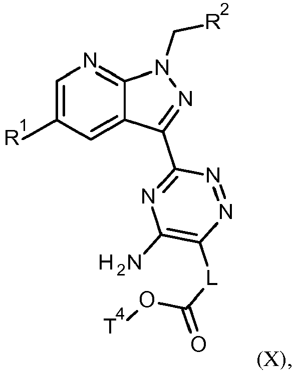
in welcher L, R¹ und R² und T⁴ jeweils die in Anspruch 1 angegebenen Bedeutungen haben,
umsetzt, und schliesslich in einem inerten Lösungsmittel gegebenenfalls in Gegenwart einer geeigneten Base zu einer Verbindung der Formel (I-B) 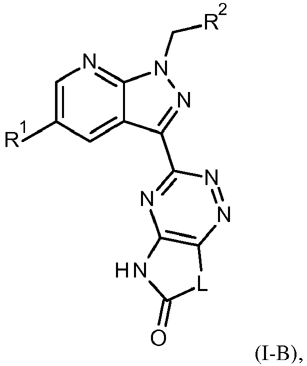
in welcher L, R¹ und R² jeweils die in Anspruch 1 angegebenen Bedeutungen haben,
cyclisiert,
und gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

3. Verbindung der Formel (I), wie in Anspruch 1 definiert, zur Behandlung und/oder Prophylaxe von Krankheiten.

4. Verbindung der Formel (I), wie in Anspruch 1 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

5. Arzneimittel enthaltend eine Verbindung der Formel (I), wie in Anspruch 1 definiert, in Kombination mit einem inerten, nicht-toxischen, pharmazeutisch geeigneten Hilfsstoff.

6. Arzneimittel enthaltend eine Verbindung der Formel (I), wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

7. Arzneimittel nach Anspruch 5 oder 6 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compounds of the formula (I-1), in which 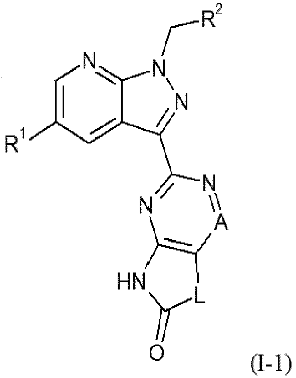 in which
A represents nitrogen or CR³,
where
R³ represents hydrogen or amino,
L represents a *-CR^{4A}R^{4B}-(CR^{5A}R^{5B})ₚ-# group,
where
* represents the point of attachment to the carbonyl group,
# represents the point of attachment to the pyrimidine ring or triazine ring,
p represents a number 0,
R^{4A} represents hydrogen, fluorine, methyl or hydroxy,
R^{4B} represents hydrogen, fluorine, trifluoromethyl, 2,2,2-trifluoroethyl or methyl,
R¹ represents hydrogen or fluorine,
R² represents thienyl, pyridyl or pyrimidinyl,
where thienyl, pyridyl and pyrimidinyl may be substituted by 1 or 2 fluorine substituents,
and the salts, solvates and solvates of the salts thereof.

2. Process for preparing compounds of the formula (I) as defined in Claim 1, **characterized in that** the compound of the formula (II) 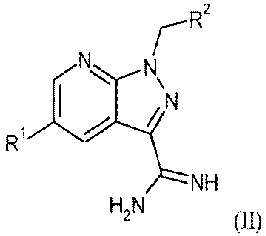 in which R¹ and R² each have the meanings given in Claim 1,
[A] is reacted in an inert solvent in the presence of a suitable base with a compound of the formula (III) 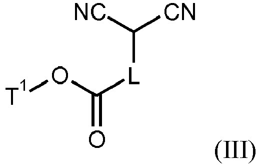
in which L has the meaning given in Claim 1 and
T¹ represents (C₁-C₄)-alkyl
to give a compound of the formula (IV) 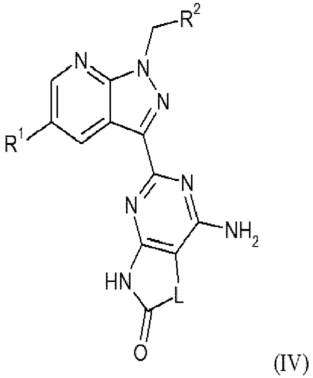
in which L, R¹ and R² each have the meanings given in Claim 1,
this is then converted with isopentyl nitrite and a halogen equivalent into a compound of the formula (V) 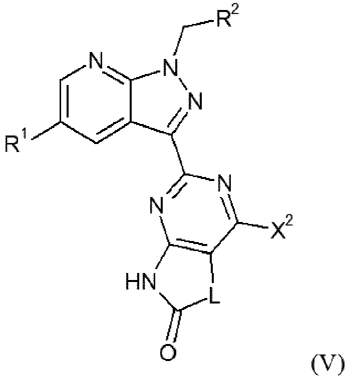
in which L, R¹ and R² each have the meanings given in Claim 1 and
X² represents bromine or iodine,
and this is then reacted in an inert solvent, in the presence of a suitable transition metal catalyst, to give a compound of the formula (I-A) 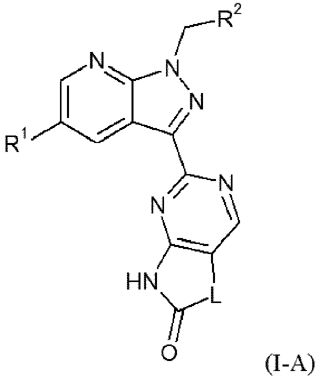
in which L, R¹ and R² each have the meanings given in Claim 1,
or
[B] is reacted in an inert solvent in the presence of a suitable base with hydrazine hydrate to give a compound of the formula (VI) 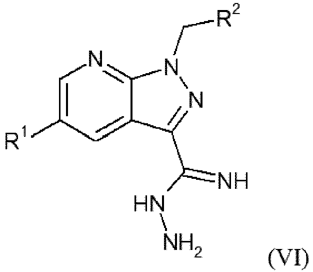
in which R¹ and R² each have the meanings given in Claim 1,
this is then reacted in an inert solvent with a compound of the formula (VII) 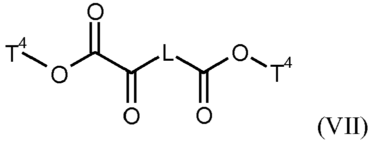
in which L has the meaning given in Claim 1 and
T⁴ represents (C₁-C₄)-alkyl
to give a compound of the formula (VIII) 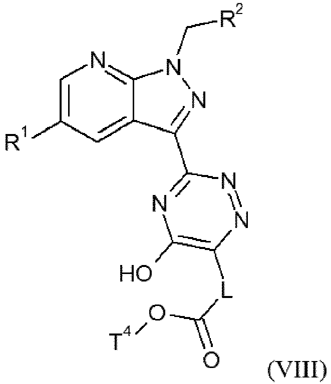
in which L, R¹ and R² and T⁴ each have the meanings given in Claim 1,
this is then converted with phosphoryl chloride into a compound of the formula (IX) 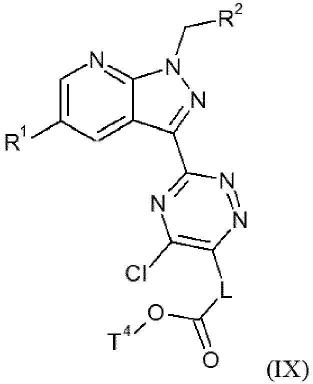
in which L, R¹ and R² and T⁴ each have the meanings given in Claim 1,
and this is reacted directly with ammonia to give a compound of the formula (X) 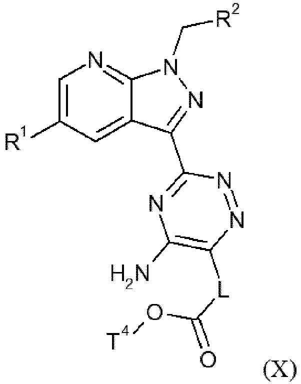
in which L, R¹ and R² and T⁴ each have the meanings given in Claim 1,
and finally cyclized in an inert solvent, optionally in the presence of a suitable base, to give a compound of the formula (I-B) 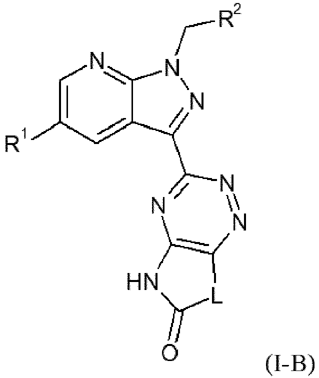
in which L, R¹ and R² each have the meanings given in Claim 1,
and the resulting compounds of the formulae (I-A) and (I-B) are, where appropriate, converted with the appropriate (i) solvents and/or (ii) acids or bases into their solvates, salts and/or solvates of the salts.

3. Compound of the formula (I) as defined in Claim 1 for the treatment and/or prophylaxis of diseases.

4. Compound of the formula (I) as defined in Claim 1 for use in a method for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

5. Medicament comprising a compound of the formula (I) as defined in Claim 1 in combination with an inert, non-toxic, pharmaceutically suitable excipient.

6. Medicament comprising a compound of the formula (I) as defined in Claim 1 in combination with a further active ingredient selected from the group consisting of organic nitrates, NO donors, cGMP-PDE inhibitors, agents having antithrombotic activity, agents lowering blood pressure, and agents altering lipid metabolism.

7. Medicament according to Claim 5 or 6 for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

## Revendications

1. Composés de formule (I-1), 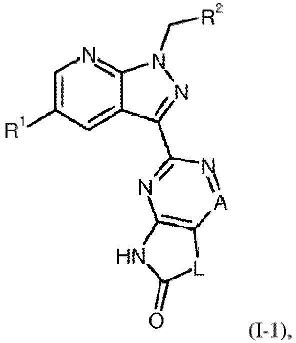 dans laquelle
A représente azote ou CR³,
R³ représentant hydrogène ou amino,
L représente un groupe *-CR^{4A}R^{4B}-(CR^{5A}R^{5B})ₚ-#,
* représentant l'emplacement de liaison au groupe carbonyle,
# représentant l'emplacement de liaison au cycle pyrimidine ou au cycle triazine,
p représentant un nombre 0,
R^{4A} représentant hydrogène, fluor, méthyle ou hydroxy,
R^{4B} représentant hydrogène, fluor, trifluorométhyle, 2,2,2-trifluoroéthyle ou méthyle,
R¹ représente hydrogène ou fluor,
R² représente thiényle, pyridyle ou pyrimidinyle,
le thiényle, le pyridyle et le pyrimidinyle pouvant être substitués avec 1 ou 2 substituants fluor, ainsi que leurs sels, solvates et solvates des sels.

2. Procédé de fabrication de composés de formule (I), tels que définis dans la revendication 1, **caractérisé en ce que** le composé de formule (II) 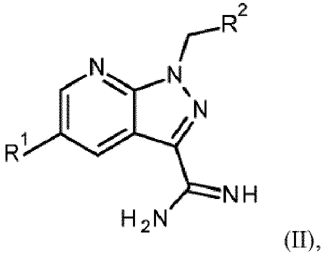 dans laquelle R¹ et R² ont chacun les significations indiquées dans la revendication 1,
[A] est mis en réaction dans un solvant inerte en présence d'une base appropriée avec un composé de formule (III) 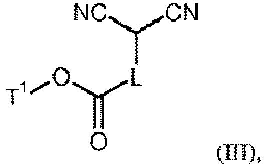
dans laquelle L a la signification indiquée dans la revendication 1, et
T¹ représente alkyle en (C₁-C₄),
pour former un composé de formule (IV) 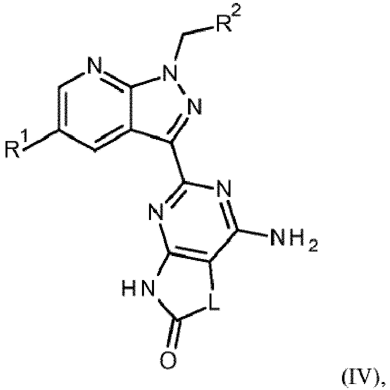
dans laquelle L, R¹ et R² ont chacun les significations indiquées dans la revendication 1,
puis celui-ci est transformé avec de l'isopentylnitrite et un équivalent d'halogène en un composé de formule (V) 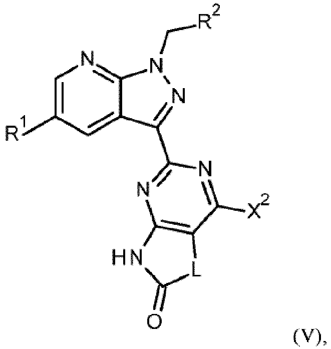
dans laquelle L, R¹ et R² ont chacun les significations indiquées dans la revendication 1, et
X² représente brome ou iode,
puis celui-ci est mis en réaction dans un solvant inerte en présence d'un catalyseur de métal de transition approprié pour former un composé de formule (I-A) 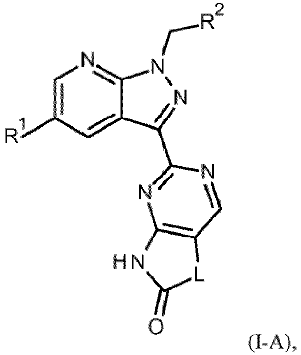
dans laquelle L, R¹ et R² ont chacun les significations indiquées dans la revendication 1,
ou
[B] est mis en réaction dans un solvant inerte en présence d'une base appropriée avec de l'hydrate d'hydrazine pour former un composé de formule (VI) 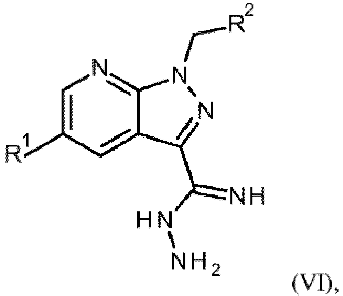
dans laquelle R¹ et R² ont chacun les significations indiquées dans la revendication 1,
puis celui-ci est mis en réaction dans un solvant inerte avec un composé de formule (VII) 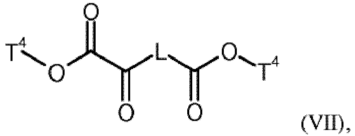
dans laquelle L a la signification indiquée dans la revendication 1, et
T⁴ représente alkyle en (C₁-C₄),
pour former un composé de formule (VIII) 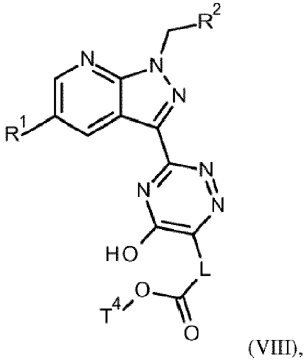
dans laquelle L, R¹ et R² et T⁴ ont chacun les significations indiquées dans la revendication 1,
puis celui-ci est transformé avec du chlorure de phosphoryle en un composé de formule (IX) 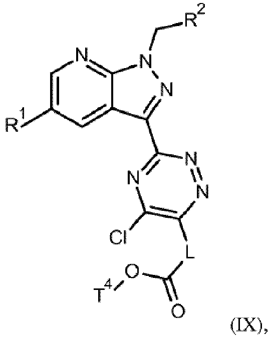
dans laquelle L, R¹ et R² et T⁴ ont chacun les significations indiquées dans la revendication 1,
et celui-ci est directement mis en réaction avec de l'ammoniac pour former un composé de formule (X) 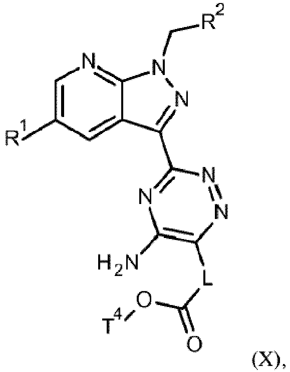
dans laquelle L, R¹ et R² et T⁴ ont chacun les significations indiquées dans la revendication 1,
puis cyclisé dans un solvant inerte, éventuellement en présence d'une base appropriée, en un composé de formule (I-B) 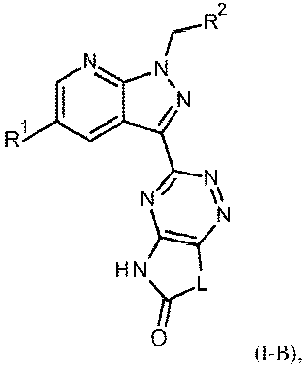
dans laquelle L, R¹ et R² ont chacun les significations indiquées dans la revendication 1,
et les composés de formule (I-A) et (I-B) résultants sont éventuellement transformés avec (i) les solvants et/ou (ii) les acides ou bases appropriés en leurs solvates, sels et/ou solvates des sels.

3. Composé de formule (I), tel que défini dans la revendication 1, pour le traitement et/ou la prophylaxie de maladies.

4. Composé de formule (I), tel que défini dans la revendication 1, destiné à une utilisation dans un procédé de traitement et/ou de prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

5. Médicament contenant un composé de formule (I), tel que défini dans la revendication 1, en combinaison avec un adjuvant inerte, non toxique, pharmaceutiquement approprié.

6. Médicament contenant un composé de formule (I), tel que défini dans la revendication 1, en combinaison avec un autre agent actif choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

7. Médicament selon la revendication 5 ou 6, destiné pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.
